(19) Europäisches Patentamt
European Patent Office
Office européen des brevets

(11) **EP 2 998 891 A1**

(12) **EUROPEAN PATENT APPLICATION**

(43) Date of publication:
**23.03.2016 Bulletin 2016/12**

(51) Int Cl.:
**G06F 19/00** *(2011.01)*

(21) Application number: **14185566.8**

(22) Date of filing: **19.09.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Technische Universität Graz**
**8010 Graz (AT)**

(72) Inventors:
• **Hartler, Jürgen**
 **8045 Graz (AT)**
• **Thallinger, Gerhard G.**
 **8010 Graz (AT)**

(74) Representative: **Dilg, Haeusler, Schindelmann Patentanwaltsgesellschaft mbH**
 **Leonrodstrasse 58**
 **80636 München (DE)**

(54) **Automated structural identification of metabolites by a generic spectra description language utilizing MSn spectra**

(57)    It is described a computer implemented method for automatically determining fragments in a sample, the method comprising: receiving a set of values of a measurement by a device, such as an mass spectrometer, of an analyte composition in the sample; determining at least one fragment that corresponds to at least one value of the set of values by processing at least one rule of a plurality of rule definitions, wherein the at least one rule defines a fragment information, and wherein the fragment information of the at least one rule defines a value that corresponds to the at least one value of the set of values; and identifying the at least one fragment by the fragment information of the at least one rule.

Fig. 1

**Description**

Field of invention

The present invention relates to the field of automated structural identification of analyte composition, in particular metabolites and/or lipids, by a generic spectra description language, a so-called fragmentation language, utilizing $MS^n$ spectra.

Art Background

Metabolites are one of the most important groups of molecules in living organisms. A method for a measurement of metabolites is mass spectrometry. Mass spectrometry has led to a rapid increase in the number, size and rate at which datasets are generated. Identifying hundreds or thousands of metabolites requires the usage of automated and reliable software tools.

Mass spectrometry offers the possibility to identify metabolites based on a mass value of an intact metabolite species. However, many metabolite species are isobaric, resulting in ambiguities about the true identity of the metabolite when the identification is based on $MS^1$ data only. $MS^n$ spectra or fragmentation spectra from complex samples carry the potential to elucidate many structural features of metabolites. In the exemplary case of lipids, $MS^n$ spectra provide information about attached fatty acids and in many cases about their regio-isomeric position. However, $MS^n$ spectra of metabolites can vary tremendously, because the fragmentation process depends on parameters like the used mass spectrometer, a fragmentation collision energy, a charge state, and adduct ions. Due to this diversity in the fragmentation process, especially the one of lipids, a generally applicable bioinformatics tool for the automated analysis of lipidomics $MS^n$ experiments is still missing.

Therefore, there may be a need for a method and/or a system for an automated identification of structural information of analyte compositions, in particular metabolites and/or lipids, based on $MS^n$ data.

Summary of the Invention

This need may be met by the subject matter according to the independent claims. Advantageous embodiments of the present invention are described by the dependent claims.

According to an aspect of the invention, there is provided a computer implemented method for automatically determining fragments in a sample. The method comprises receiving a set of values of a measurement by a device, such as a mass spectrometer, of an analyte composition, e.g. metabolites and/or lipids, in a sample. And, determining at least one fragment that corresponds to at least one value of the set of values by processing at least one rule of a plurality of rule definitions, wherein the at least one rule defines a fragment information, and wherein the fragment information of the at least one rule defines a value, e.g. an m/z value, that corresponds to the at least one value of the set of values. Then, the fragment is identified by the fragment information of the at least one rule. For example, one rule may provide one fragment information, and one fragment information may refer to one fragment to be determined. This may provide the advantage that fragments may be defined more flexibly and/or more easily, since the determining of a fragment may be based only on the defined rules. Any changes or adaptations to rule definitions may directly influence the determining of the at least one fragment.

According to a further embodiment of the invention, at least one rule may be defined for each fragment in the plurality of rule definitions. This may provide the advantage that each fragment may be determined separately from another fragment. Thus, a more flexible determination of fragments may be achieved.

According to a further embodiment of the invention, the set of values of the measurement may correspond to $MS^n$ spectra of the analyte composition, and/or the at least one fragment is identified in a first spectrum of the $MS^n$ spectra and a different fragment is identified in a different spectrum of the $MS^n$ spectra. This may provide the advantage that multiple mass spectra of the analyte composition may be used to determine the fragments of the analyte composition. Thus, a more flexible and/or more generic application of the rules may be achieved. In particular, the rules may be applied to any one of the $MS^n$ spectra, i.e. the rules may be used independently of the number of spectra available. Accordingly, a spectrum may be searched which includes the at least one fragment.

According to a further embodiment of the invention, the analyte composition may comprise at least one analyte species, e.g. phosphatidylinositol, triacylglycerol, phosphatidylcholin, and/or 1-alkyl,2-acylglycerophosphoethanolamines. This may provide the advantage that the method and, in particular the rules, may be applied in a flexible manner to different analyte classes.

According to a further embodiment of the invention, the set of rules may comprise at least one rule of a first type and at least one rule of a second type, wherein the at least one rule of the first type may define fragment information, and the at least one of rule of the second type may define intensity information. This may provide the advantage that the specificity of the determining may be increased. Further, more complex rules may be defined in a simple manner by using only two different types of rules.

According to a further embodiment of the invention, the processing of at least one rule of the first type may comprise determining at least one of a head fragment and/or a chain fragment based on the fragment information. This may provide the advantage that the fragment information may be easily adapted to different fragment types using a single rule type, i.e. the first rule type.

According to a further embodiment of the invention, the processing of at least one rule of the second type may comprise checking whether at least one rule of the second type is defined. If at least one rule of the second type is defined, it may be determined whether the intensity information of the at least one rule of the second type is fulfilled by the set of values of the measurement to verify at least one of a head fragment, a chain fragment, and/or a position of the chain fragment. And, the at least one fragment may be identified by the fragment information of the at least one rule of the first type and the intensity information of the at least one rule of the second type. This may provide the advantage that the verification and/or the determining of a fragment may be efficiently improved by evaluating the intensity information. In particular, a more accurate determination of the fragment may be enabled and/or a probability of determining false positives may be reduced.

According to a further embodiment of the invention, the intensity information may be defined relative to one or more fragments as defined by the fragment information of the at least one rule of the first type. This may provide the advantage that fragment information of the first rule type may be easily used for defining a rule of the second rule type. Further, the relative definition of intensity information may allow for a customizable pattern which may be applied context sensitive. Thus, a more flexible and easy to implement intensity information for fragments may be enabled in an efficient manner.

According to a further embodiment of the invention, the plurality of rule definitions may define at least one rule for at least one of a head fragment, a fatty acid chain, and/or a position of a fatty acids chain of the analyte. This may provide the advantage that different fragments of the analyte may be processed separately, e.g. sequentially and/or in parallel. For example, if the plurality of rule definitions are processed sequentially, the at least one rule of the head fragment may be processed first. Second, the at least of rule of the fatty acid chain, and third, the at least one rule of the position of the fatty acid chain may be processed. Thus, a more accurate and more flexible determining of fragments may be enabled.

According to a further embodiment of the invention, the at least one rule may define at least one fragment of the analyte relative to a placeholder, wherein the placeholder may comprise at least one of a precursor, an acyl chain, an alkyl chain, and/or an alkenyl chain. This may provide the advantage that the implementation of the rule may be simplified. Further, patterns to determine fragments of the at least one rule may be implemented in a simplified manner.

According to a further embodiment of the invention, the at least one rule may comprise a property to define whether the at least one rule is mandatory. This may provide the advantage that a minimum set of rules, i.e. the mandatory rules, may be easily defined. In particular, non-mandatory rules may be used to further increase the confidence in the determined fragment. Thus, the reliability of the determining of the at least one fragment and the derivable information therof may be efficiently increased.

According to a further embodiment of the invention, the method may further comprise filtering the set of values based on a predefined threshold value prior to determining the at least one fragment. This may provide the advantage that noisy measurement values may be removed and/or filtered in a simplified manner.

According to a further embodiment of the invention, the method may further comprise providing structural information of the analyte regarding a head fragment, one or more fatty acid chains, and/or a positional information of the one or more fatty acid chains as a result. This may provide the advantage that an output is provided which comprises all information which has been determined for the analyte based in the determined fragments.

According to a further aspect of the invention, there is provided a computer system for automatically determining fragments in a sample. The computer system comprises a memory configured to store computer-readable instructions, and a processor configured to process the computer-readable instructions to perform the method as described above.

According to a further aspect of the invention, there is provided a computer-readable medium, the computer-readable medium comprising instructions which, when executed on a computer system, perform the method as described above.

The instructions may be implemented as a computer readable instruction code in any suitable programming language, such as, for example, JAVA, C++, and may be stored on a computer-readable medium (removable disk, volatile or nonvolatile memory, embedded memory/processor, etc.). The instruction code is operable to program a computer or any other programmable device to carry out the intended functions. The computer readable instruction code may be available from a network, such as the World Wide Web, from which it may be downloaded.

The invention may be realized by a computer program respectively software module. However, the invention may also be realized by one or more specific electronic circuits respectively hardware. Furthermore, the invention may also be realized in a hybrid form, i.e. in a combination of software modules and hardware modules.

It has to be noted that embodiments of the invention have been described with reference to different subject matters. In particular, some embodiments have been described with reference to method type claims whereas other embodiments have been described with reference to apparatus type claims. However, a person skilled in the art will gather from the above and the following description that, unless other notified, in addition to any combination of features belonging to one type of subject matter also any combination between features relating to different subject matters, in particular between features of the method type claims and features of the apparatus type claims is considered as to be disclosed with this document.

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.

Brief Description of the Drawing

**Figure 1** illustrates exemplary multi-stage mass spectrometry, short $MS^n$, spectrum 100 of a lipid.

**Figure 2** illustrates an overview of an algorithm for searching and/or finding one or more attached fatty acid chains.

**Figure 3** illustrates an overview of a searching and/or finding process for a positional assignment of fatty acids to a backbone.

**Figure 4** shows an exemplary spectrum of TG 16:0_18:1_18:0

**Figure 5** shows exemplary fragmentation spectra of a lipid class PC.

**Figure 6** shows an $MS^1$ chromatogram, and an $MS^2$ spectrum of an identified $MS^1$ peak.

**Figure 7** illustrates an overlap of regio-isomeric PC 16:0/18:1 and PC 18:1/16:0 at equal intensities in the form of chromatograms.

**Figure 8** shows an exemplary list of parameters of a "GENERAL" section.

**Figure 9** presents a code example of a "HEAD" section, and its derived fragments.

**Figure 10** shows an exemplary determination of a mandatory head fragment.

**Figure 11** shows an overview of a processing of intensity rules (type 2) of a "HEAD" section.

**Figure 12** illustrates a generation of possible chain combinations.

**Figure 13** shows an overview of rules of type 1 related to chains.

**Figure 14** shows an exemplary processing of rules of type 1 to determine a chain fragment.

**Figure 15** shows a processing of intensity rules (type 2) of a "CHAINS" section.

**Figure 16** shows a processing of rules of type 2 of a "POSITION" section.

Detailed Description

The illustration in the drawing is schematically. It is noted that in different figures, similar or identical elements are provided with the same reference signs or with reference signs, which are different from the corresponding reference signs only within the first digit.

In the following, an algorithm for an identification of structural information of analytes, in particular metabolites, is described. Further, a rule-based language for the formal description of $MS^n$ spectra of metabolites is presented. This language provides one or more rules to define observable fragments and relative intensities thereof. The algorithm may interpret this language and may derive structural information about metabolites based on these rules. In other words, the algorithm is based on user-definable rules for a theoretical description of an appearance of $MS^n$ spectra. One or more customizable rules may allow for a description of fragment peaks, and of their intensity relationship. Particularly, the definition of rules for the intensity relationship may be important, since intensity information reveal e.g. regio-specific information, i.e. a position of fatty acids at a backbone, and may be invaluable quality criteria for a correctness of a match. These rules may be easily defined and changed by users, since they can be directly visually controlled based on experimental spectra. By this generic approach, the algorithm might not depend on a specific database, but may be easily extended to other metabolite classes and/or different experimental conditions. In the case of lipids, the algorithm may allow to define rules for the verification of a lipid class, attached fatty acid moieties or chains, and for a regio-isomeric position of the fatty acid chains at a backbone, i.e. a backbone molecule.

The algorithm may provide the following advantages: high flexibility, higher specificity, and easy customizability. In particular, the decision for a good match is based completely on the defined rules and may be easily changed. The user may specify the fragments, where they are observed, which confidence they should have, and/or their relative intensities to one another. In this way, the confidence may be defined for each lipid class/adduct combination separately.

A quantitation and an identification of an intact metabolite species on the $MS^1$ level may work as described in the patent application US2013126725. The highly specific $MS^1$ algorithm in combination with the high specificity of the $MS^n$ algorithm presented in this application may allow for a highly specific and sensitive automated identification of lipid species, even in a presence of isobaric substances. The data may originate from biological samples, such as for example murine hepatocytes and adipocytes from a goat.

Liquid chromatography in combination with mass spectrometry, so-called LC-MS, may measure quantitative changes of hundreds of lipids simultaneously. However, many lipid species may be isobaric, resulting in ambiguities about the true identity of the lipid when the identification is based on $MS^1$ data only. $MS^n$ spectra (or fragmentation spectra) may provide the potential to elucidate many structural features of lipids, such as a prove for the lipid class, for the attached fatty acids and in many cases for the regio-isomeric position. However, $MS^n$ spectra of lipids may vary tremendously, because the fragmentation process depends on parameters like the used mass spectrometer, the collision energy, the charge state, and the adduct ions. Accordingly, a flexible and automated identification of structural information of analytes is performed based on $MS^n$ data.

The description of spectral fragments and acceptance of hits by a rule-based language may provide the following advantages:

a) the possibility to define or remove fragments based on simple rules without changing or modifying a database,

b) the absence of fragments can, but does not need to be exclusive, i.e. it is definable whether fragments are mandatory; if fragments are defined to be not mandatory, they may be used to raise the confidence if they are detected, but their absence is not exclusive, and

c) relative intensity information may be easily integrated in the rules.

Therefore, a generic solution for the structural identification of metabolites may be provided by the algorithm and the fragmentation language which may have the advantages as described above.

**Definitions**

The technical terms and expressions used within the scope of this application are generally to be given the meaning commonly applied to them in the pertinent arts of bioinformatics and mass spectrometry.

The term "subject" may relate to a plant, an animal, a human, eukaryotic and/or prokaryotic cells and/or complex biological mixtures. Further, the term "subject" may refer to any warm-blooded animal, particularly including a member of the class mammalia such as, without limitation, humans and non-human primates such as chimpanzees and other apes and monkey species; farm animals such as cattle, sheep, pigs, goats and horses; domestic mammals such as dogs and cats; laboratory animals including rodents such as mice, rats and guinea pigs, and the like. The term "subject" might not denote a particular age or sex and, thus, includes adult and newborn subjects, whether male or female.

The term "mass spectrometer", short MS, may refer to a device that measures a parameter that may be translated into mass-to-charge ratios of ions. Mass spectrometers may generally include an ion source and a mass analyzer. Examples of mass spectrometers are a time-of-flight, a magnetic sector, a quadrupole filter, an ion trap, an ion cyclotron resonance, an electrostatic sector analyzer and/or hybrids of these. Mass spectrometry may refer to the use of a mass spectrometer to detect gas phase and/or liquid phase ions.

The term "normal subjects" may relate to a subject as defined above not suffering under a disease.

The term "sample" as used herein may refer to a biological sample, meaning a sample obtained from a subject or from food and crude oil extracts. The biological sample may be selected, without limitation, from a group comprising blood, plasma, serum, sweat, and/or saliva, and/or including sputum, urine, and/or the like. As used herein, serum may refer to a fluid portion of the blood obtained after removal of a fibrin clot and/or blood cells, and/or distinguished from the plasma in a circulating blood. As used herein, plasma may refer to a fluid, non-cellular portion of the blood, and/or distinguished from the serum obtained after a coagulation.

The term "PI" may refer to a standardized abbreviation for phosphatidylinositol (see e.g. Liebisch et al.: Lipid-Maps, Journal of Lipid Research, 54(6):1523-30), "TG" to an abbreviation for triacylglycerol, "PC" to an abbreviation for phosphatidylcholine, and/or "PE" to an abbreviation for phosphatidylethanolamine. As used herein, the term "PI" and/or any of the other abbreviations may be used with a notation "X:Y", for example "38:4", where "X" may be the amount of carbon atoms in one or more attached fatty acid chains, and "Y" may be the amount of zero, one or more double bonds. In the case of PI, two fatty acid chains may be attached to a glycerol backbone. If "PI" is used with the notation "A:B_C:D", for example PI 18:0_20:4, a stereospecific position of the fatty acid chains A:B and/or C:D may be unknown. A symbol "A" may refer to the amount of carbon atoms in a fatty acid chain, and a symbol "B" may refer to the amount of double bonds of this fatty acid chain. Further, symbols "C" and "D" may correspond to the same notation for another fatty acid chain. If "PI" is used with the notation "A:B/C:D", the fatty acid chain "A: B" may be attached to the first available stereospecific position of the glycerol backbone, and "C: D" at the second available stereospecific position of the glycerol backbone.

The term "sn-X", such as "sn-1", may correspond to a stereospecific numbering which may reflect a position of the fatty acid chain at a lipid backbone such as the glycerol backbone, where "X" may be a stereospecific number.

The term "spectrum intensity coverage" may specify a relative amount of total intensities that must be covered by found peaks assignable to defined fragments.

The term "analyte" is a collective term comprising all molecules in a sample having the same chemical composition and chemical structure, including their isotopes.

The term "intensity" refers to a quantitative measure for the amount of molecules entering a mass detector at a specific m/z, and time.

The present invention is further described by reference to the following nonlimiting figures and examples:

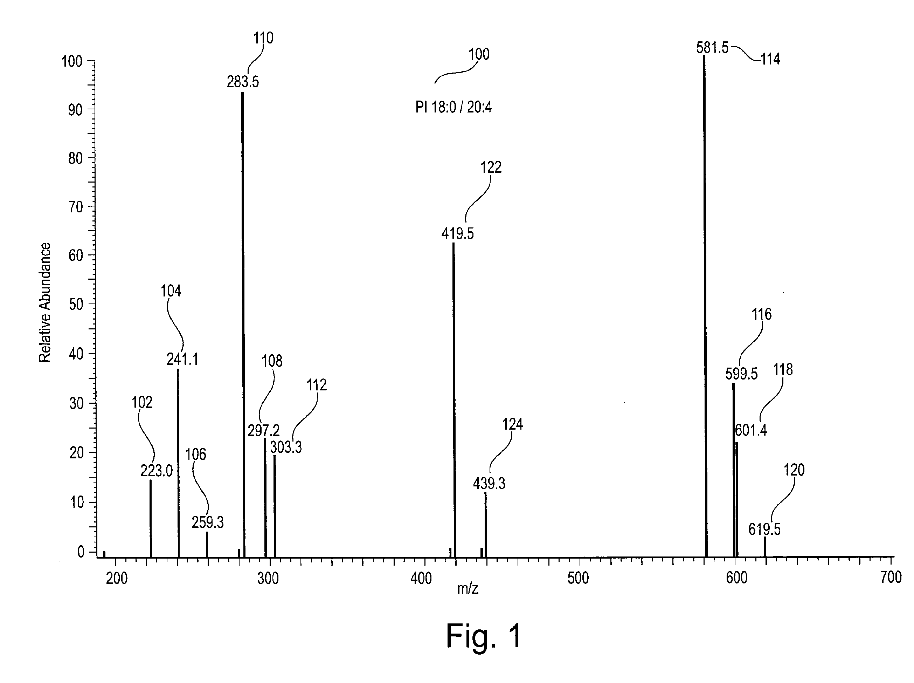

**Fig. 1** illustrates exemplary multi-stage mass spectrometry $MS^n$ spectrum 100 of a lipid. The symbol "n" of the term "$MS^n$" refers to the number of ion stages, e.g. 1, 2, 3, etc. An MS/MS spectrum, i.e. a mass spectrum obtained using e.g. a tandem mass spectrometry, of m/z 855.55 which may correspond to an m/z of an intact PI 38:4 (sn-1: 18:0; sn-2: 20:4; sn-3: PI head group). The term "m/z" represents a quantity in Thompson [Th] formed by dividing a ratio of the mass of an fragment to a uniformed atomic mass unit in Dalton [Da] by its number of charges (regardless of sign). As depicted in Fig. 1, fragments 102, 104, 106, and 108 may be formed from the head group, and as such the fragments 102 to 108 may corroborate that PI 38:4 may be of a lipid class PI. The fragments 102, 104, and 106 may correspond to an inositol phosphate and losses of $H_2O$ thereof. The fragment 108 may represent an inositol phosphate with the glycerol backbone attached. Fragments 110, 112, 114, 116, 118, 120, 122, and 124 may be formed of attached fatty acid chains and/or losses thereof, respectively. Fragments that may indicate an existence of a fatty acid 18:0 may be fragments 110, 118, 120, and 124; fragments that may indicate an existence of a fatty acid 20:4 may be 112, 114, 116, and 122. The fragments 110 and 112 may be carboxylates of the fatty acids 18:0 and 20:4. The fragment cluster 114, 116, 118, and 120 may represent neutral losses of the fatty acid 20:4 and the fatty acid 18:0, either as fatty acids, the fragments 114 and 118, or as ketenes, the fragments 116 and 120. Finally, peaks of fragments 122 and 124 may derive from a neutral loss of either the fatty acid 20:4 and/or the fatty acid 18:0 followed by an additional loss of an inositol from the head group. The intensity of fragment pairs, e.g. the fragments 114 and 118, may be specific for a location of a stereospecific position. According to fragment intensities of the mass spectrum of Fig. 1, an observed lipid may be PI 18:0/20:4 and not PI 20:4/18:0.

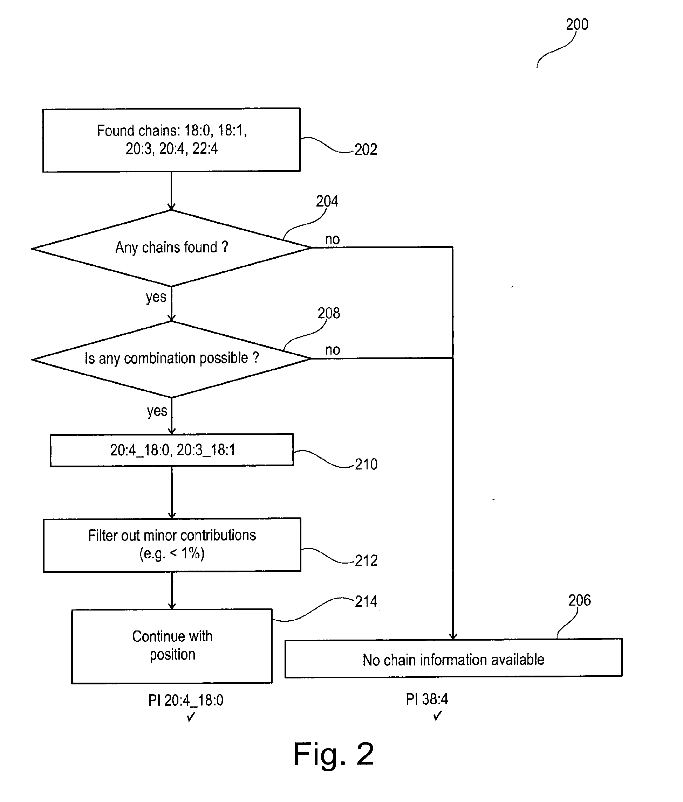

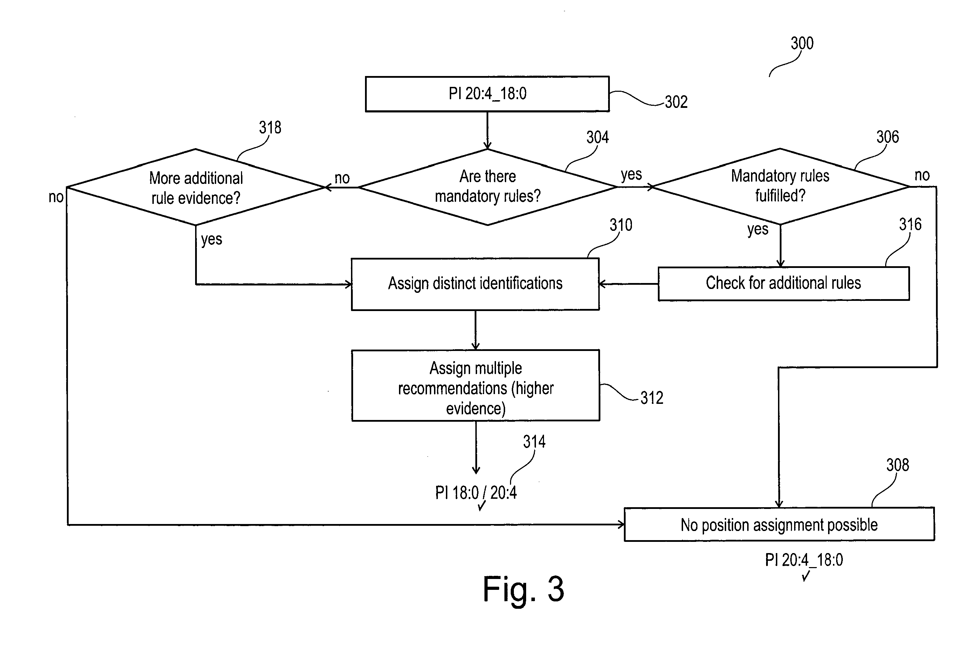

**Fig. 2** illustrates an overview of an algorithm for searching and/or finding process 200 for one or more attached fatty acid chains by previously detected fatty acid chain fragments. In particular, fragments may be observed and/or found for the fatty acid chains 18:0, 18:1, 20:3, 20:4, 22:4 for the lipid species PI 38:4. The algorithm may check 204 if there are any possible chains which may be verified by one or more rules. If there are no possible chains which may be verified by the one or more rules, the algorithm may stop and may declare 206, e.g. provide an output, that there is no information about chains available. Next, the algorithm may check 208 if any combination of the observed/found fragments, e.g. observed/found fragments 18:0, 18:1, 20:3, 20:4, 22:4, may correspond to the number of carbon atoms and the number of double bonds of the attached fatty acids, e.g. the lipid species PI 38:4. In this example, the fatty acid 22:4 may be discarded since a required 16:0 fragment has not been found, i.e. did not pass the fragmentation rules. Thus, PI 38:4 may comprise two isobaric species 210, namely 18:0_20:4 and 18:1_20:3. Next, one or more species, e.g. one or more species of the isobaric species 18:0_20:4 and/or 18:1_20:3, may be removed or filtered out 212 that may contribute only to a minor extend to the total intensity. For this optional check, all fragment intensities of one combination, e.g. combination 18:0_20:4 and/or 18:1_20:3, may be summed to yield a total intensity. If the intensity of one combination is lower than a threshold relative to the most intense combination, e.g. the intensity of one combination is lower than 1% to the most intense combination, the identification may be discarded and/or filtered out. In this example, PI 38:4 is composed of the fatty acids 18:0 and 20:4. Here, only one possible chain combination is reported as result, but the algorithm may return several chain combinations, since MS$^n$ data of biological samples usually comprises more than one species. Next, a positional assignment for the fatty acids, e.g. the fatty acids 20:4 and 18:0, within PI 38:4 may be determined 214. This process will be described with respect to Fig. 3.

**Fig. 3** illustrates an overview of a searching and/or finding process 300 for a positional assignment of fatty acids to a backbone. As an input, the process may receive 302 one or more fatty acid combinations of a lipid species, e.g. the fatty acid combination 20:4 and 18:0 of the lipid species PI 38:4, noted as PI 20:4_18:0. Next, it is checked 304 whether there are mandatory rules which have to be fulfilled for finding the positional assignment of the received fatty acids. If one or more mandatory rules are present, all of them must be fulfilled 306, except those where referenced fragments are not present, e.g. fragments which are discarded or filtered out as described with respect to the process of Fig. 2. Rules which are not mandatory, i.e. rules including an attribute "mandatory=false", may be checked 316 for additional evidence only. However, the rules which are not mandatory might not be decisive for finding the position of the fatty acids. If no mandatory rules are present, the algorithm may determine 318 the position assignment based on a majority of matching rules. If no majority of matching non-mandatory rules can be determined and/or the mandatory rules cannot be fulfilled, the algorithm might not perform a position assignment, i.e. the position assignment might not be possible 308. Otherwise, i.e. if a majority of matching non-mandatory rules can be determined and/or the mandatory rules can be fulfilled, the fatty acid position is assigned as follows:

- the algorithm may assign 310 fatty acids to one or more positions where there is no evidence of other fatty acids for this position, so-called distinct matches.

- the algorithm may assign 312 fatty acids to one or more positions where more than one fatty acid may match, which can be the case with more than two fatty acids. In particular, the fatty acid for which more rules match for this position may be assigned.

The result of the position assignment may be provided 314 as output. For example, an output PI 18:0/20:4 may define that there are two fatty acid positions. For the first fatty acid position, the fatty acid 18:0 is determined and, for the second fatty acid position, the fatty acid 20:4 is determined.

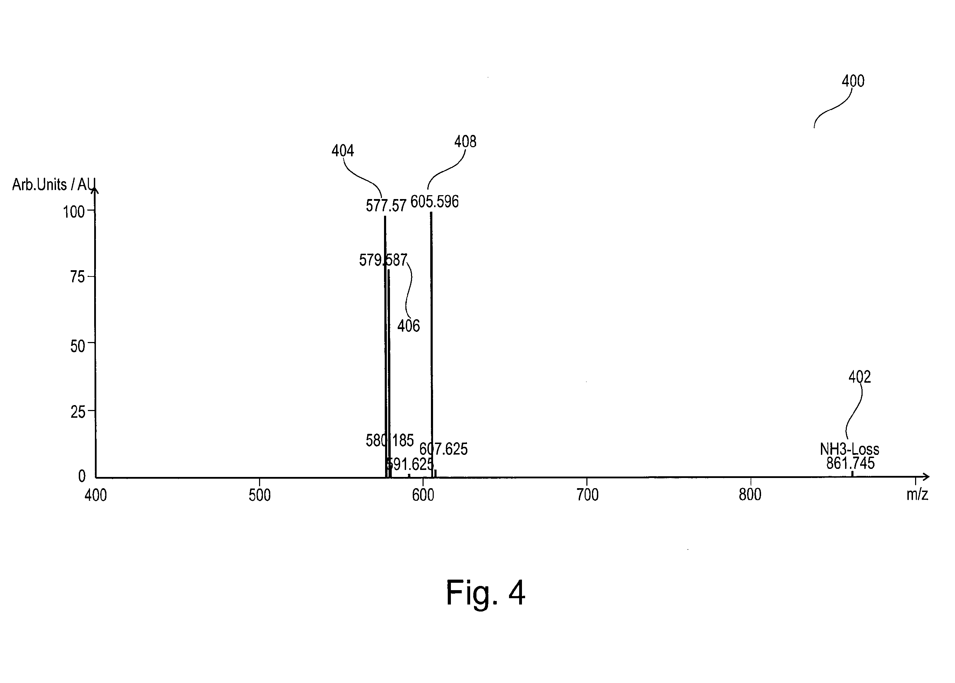

**Fig. 4** shows a spectrum 400 of TG 16:0_18:1_18:0. The spectrum may comprise a small fragment at 862 Th 402, i.e. an NH3-Loss, which may be characteristic for the TG class but this fragment might not be observed frequently. The neutral losses of the fatty acid moieties may be prominent peaks in the spectrum, e.g. 577 Th 404, 579 Th 406, and/or 605 Th 408. Thus, the spectrum intensity coverage may be used as quality criterion for a matching. Furthermore, for a TG species, a complete stereospecific assignment based on fragmentation spectra might not be possible, since losses at sn-1 and sn-3 may occur approximately at the same frequency (see e.g. Hsu FF and Turk J, American Oil Chemists Society Press; 2005:61-179). In particular, only losses at sn-2 may be less prominent. Thus, only NL Carboxy 18:1 406 may be assigned to sn-2 position.

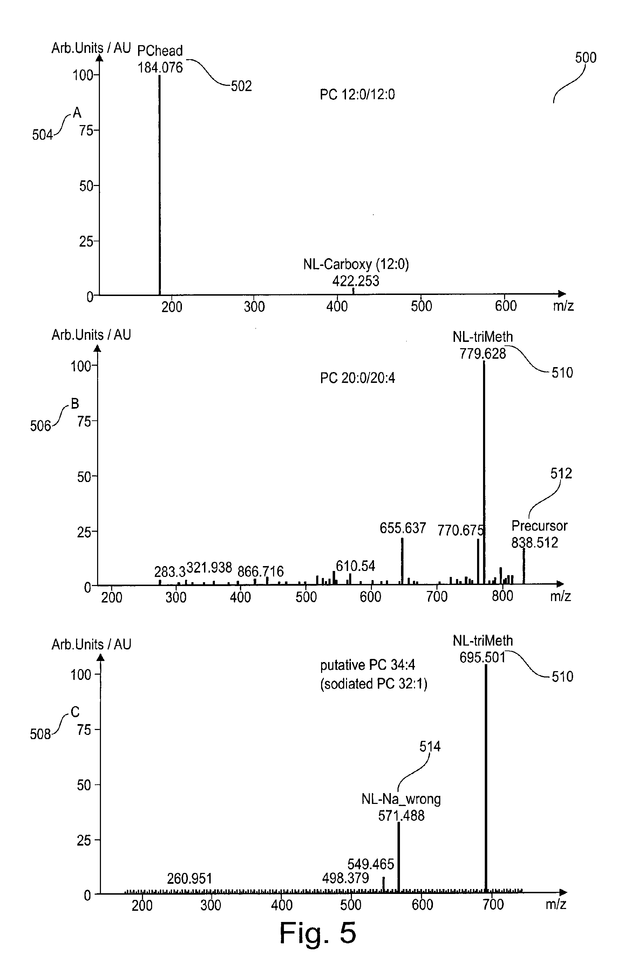

**Fig. 5** shows fragmentation spectra 500 of a lipid class PC. Typically, PC species may be protonated in a positive ion mode and may show a very strong intensity for an ionized head group 502 at m/z 184 Th. A first diagram (A) 504 shows a fragmentation spectrum of PC 12:0/12:0 which may clearly exhibit the head group fragment 502. A second diagram (B) 506 may show a fragmentation spectrum of PC 20:0/20:4. The PC head group fragment may be missed because of an automated change in a scanned mass range by a mass spectrometer. The PC 20:0/20:4 has a higher molecular weight than PC 12:0/12:0, and the mass spectrometer may automatically adjust to a higher range for the fragmentation spectrum, excluding the m/z of 184 Th from the analysis. However, for these masses a precursor 512 and a neutral loss of $NC_3H_9$ can be observed labelled "NL-triMeth" 510 in the second diagram. A third diagram (C) 508 may represent a putative fragmentation spectrum of a protonated PC 34:4, which may exhibit the "NL-triMeth" fragment 510, but not the head fragment 502 of 184 Th, although this fragment would be within the scan range. The reason may be that this is actually the fragmentation spectrum of the isobar sodiated form of PC 32:1 which is formed in addition to the protonated form in the ion source, but at lower amounts.

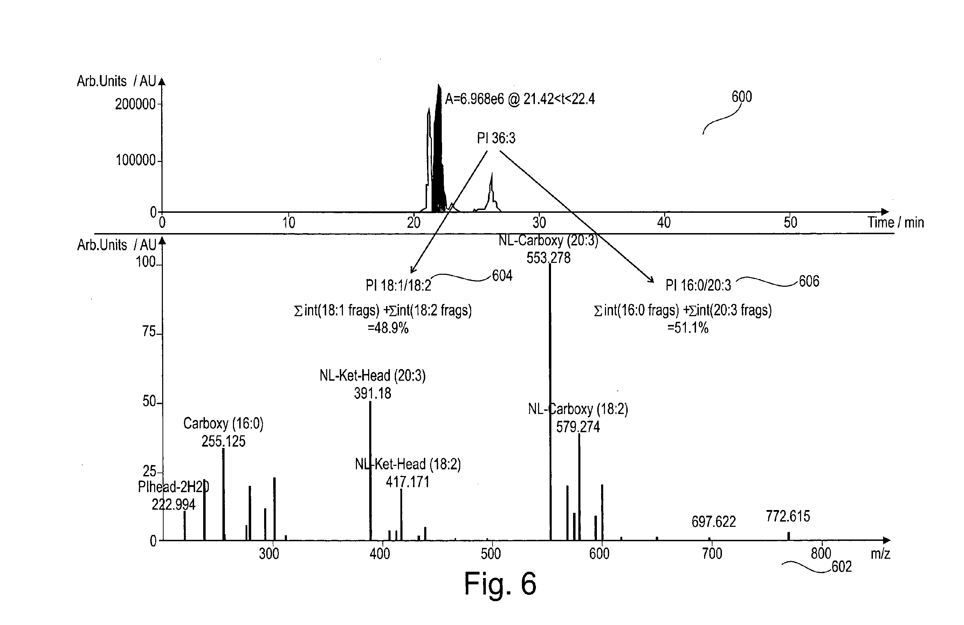

**Fig. 6** shows an MS$^1$ chromatogram 600 where PI 36:3 has been identified. The corresponding fragmentation spectrum 602 at the bottom may reveal this peak to comprise PI 18:1/18:2 604 and PI 16:0/20:3 606. The algorithm may determine a sum of intensities of fragments belonging to each of the chain combination, i.e. a sum of fragments revealing 18:1 and 18:2 for PI 18:1/18:2 and a sum of fragments revealing 16:0 and 20:3 for PI 16:0/20:3. The intensity determined by MS$^1$ may be split among both species corresponding to these intensity proportions.

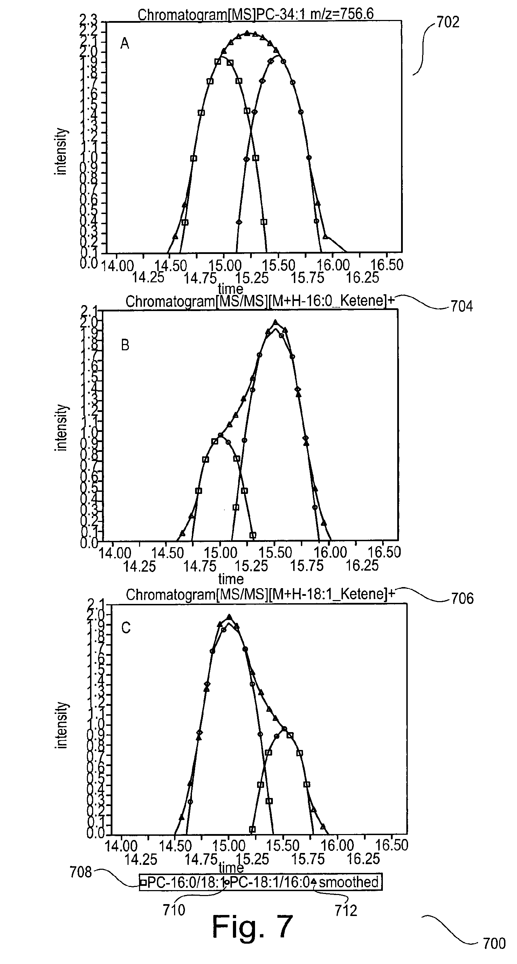

**Fig. 7** illustrates an overlap of regio-isomeric PC 16:0/18:1 and PC 18:1/16:0 at equal intensities in the form of chromatograms 700. A first diagram (A) 702 shows an MS$^1$ chromatogram, a second diagram (B) 704 shows an MS$^2$ chromatogram of a loss of 16:0 as ketene, and a third diagram (C) 706 shows an MS$^2$ chromatogram of the loss of 18:1 as ketene. A contribution of PC 16:0/18:1 is depicted with square points 708, a contribution of PC 18:1/16:0 with circle points 710. A smoothed sum signal of both contributions, the contribution of PC 16:0/18:1 and the contribution of PC 18:1/16:0, is depicted with triangle points 712. In the first diagram 702, it might not be possible to differentiate between the two analytes 708 and 710. However, the MS/MS signals in the second diagram 704 and the third diagram 706 have contrary peak shapes with peak summits at different retention times. If there is a sufficient number of measurements, separation of such peaks may be possible. It might be possible that only two $MS^n$ spectra allow for a prediction of a presence of one or two regio-isomeric species. If both regio-specific fragments behave in the same manner (i.e. both intensities rise or fall), the presence of just one or a dominant species may be assumed. If the intensities behave contrarily, a presence of both species must be assumed.

**EXAMPLE 1: fragmentation language for the example of PI (phosphatidylinositol) in negative ion mode**

$MS^n$ spectra may vary considerably depending on a setup of an MS device. In order to support different setups, a rule-based language, so-called fragmentation language FL, is presented which may enable a formal description of $MS^n$ spectra, establish one or more rules to classify spectra, and differentiate between potential candidates. Further, an algorithm is provided which processes the one or more rules to classify the spectra. In the following, it is explained how structural information may be obtained from $MS^n$ spectra using the exemplary lipid class of phosphatidylinositol PI. Additionally, an example of the fragmentation language is described for the exemplary lipid class PI.

In the case of lipids, for most lipid classes, at least three different types of information may be deduced from an $MS^n$ spectrum: 1) information about the lipid class; 2) information about the fatty acids the lipid consists of; and 3) information about the stereo-specific position of the fatty acids at the backbone. Additionally and/or alternatively, information about a position of double bonds may also be deducted from a $MS^n$ spectrum. A deduction of such information is exemplified in Figure 1 for PI in negative ion mode and a loss of hydrogen. In order to fully support the deduction of such information, the proposed rule-based language must be able to define fragments, and intensity relationships thereof. Accordingly, the proposed rule-based language may comprise two rule types: (i) rules that may define fragments, and (ii) rules that may define relative intensity relations thereof.

Rules for a fragment definition may comprise the following properties:

- Name: arbitrary name of the fragment, such as "PIhead"; the name of the fragment may be a unique name which may be used as an identifier of a rule

- Formula: description of the elemental composition of the element; the formula may comprise a chemical formula, such as $C6H12O9P$, or placeholders such as $PRECURSOR, or of arbitrary names of previously defined fragments, or any additive or subtractive combination thereof. Non-exclusive placeholders are $PRECURSOR for an elemental composition of the intact $MS^1$ molecule, and $CHAIN for an elemental composition of any fatty acid chain.

- Charge: the charge which the fragment will have

- MSLevel: the $MS^n$ level the fragment is likely to be observed, where n corresponds to the MS level

- mandatory: true if the rule is exclusive if not fulfilled; false if it adds evidence if fulfilled, but does not exclude the information to be possible

A code example for a rule for an element comprising a chemical formula only may comprise:

*Name=PIhead Formula=C6H12O9P Charge=1 MSLevel=2 mandatory=false.*

A code example for a rule representing the neutral loss of a fatty acid chain using placeholders may comprise:

*Name=NL-Ketene Formula=$PRECURSOR-$CHAIN+H2O Charge=1*
*MSLevel=2 mandatory=false.*

A code example for a rule using a defined fragment of a previous rule may comprise:

*Name=NL-Ket-Head Formula=NL-Ketene-C6H12O6 Charge=1 MSLevel=2 mandatory=false.*

Rules for a relative intensity relation comprising the following properties or attributes:

- Equation: This property describes an intensity relation between fragments, e.g. fragments which have been found according to one or more fragment definitions; the equation may comprise a smaller than symbol "<", or a greater than symbol ">". The equation may allow for multiplicative changes of the intensity values of one or more fragments, e.g. one or more fragments which have been found according to one or more fragment definitions. The equation must comprise a reference to one or more previously defined fragments and may include a position of one or more fragments, and/or a placeholder $BASEPEAK for the highest intensity found in the spectrum. For example, a name property of a previously defined fragment may be used as the reference.

- Mandatory: This property may comprise a binary value, e.g. true or false. A property value true defines that a rule has to be fulfilled, i.e. the rule is exclusive if the rule is not fulfilled. A property value false defines that a rule may be fulfilled. If a rule with a property value false is fulfilled, the rule may add additional evidence for identifying a fragment. If a rule with a property value false is not fulfilled, the result of the rule might not be considered for identifying a fragment.

A code example for a rule using defined fragments may comprise:

*Equation=PIhead-H2O>2\*PIhead mandatory=true.*

The code example may specify that the PIhead-H2O intensity must be at least twice the PIhead intensity.
A code example for a rule using defined fragments and a $BASEPEAK placeholder may comprise:

*Equation=PIhead-H2O>0.1\*$BASEPEAK mandatory=true.*

The code example may specify that the PIhead-H2O intensity must be at least 10% of the highest intensity in the spectrum.
A code example for a rule using a position attribute may comprise:

*Equation=NL-Ketene[2]\*0.7>NL-Ketene[1] mandatory=true.*

The code example may specify that the NL-Ketene fragment at position 2 times 0.7 must be bigger than the NL-Ketene fragment at position 1.
These rules may allow for a partial, in particular a complete, description of $MS^n$ fragments and its relative intensities to each other. In the following example, example 2, an algorithm for processing the rules is described in detail.

**EXAMPLE 2: fragmentation language interpretation and decision finding at the example of PI (phosphatidylinositol) in negative ion mode**

In this example, a complete set of rules for PI is described based on an exemplary spectrum as shown in Fig. 1. The example may show how structural information is obtained using fragmentation rules. For this example, three types of structural information may be obtained:

- structural information about a head group for a verification of a lipid class,

- structural information about one or more attached fatty acids in the form of number of carbon atoms and double bonds, and/or

- structural information about one or more sn positions of the one or more attached fatty acids.

Accordingly, a set of rules which may be stored in a file and/or a database, may be divided in three subsections, namely "HEAD", "CHAINS", and "POSITION". At least one or each of these sections may comprise rules for a definition of one or more fragments and/or an intensity relationship between one or more fragments. Additionally, a general section "GENERAL" may be defined to provide general information about an analyte class under observation. Except for the "GENERAL" section, none of the other sections may be mandatory.

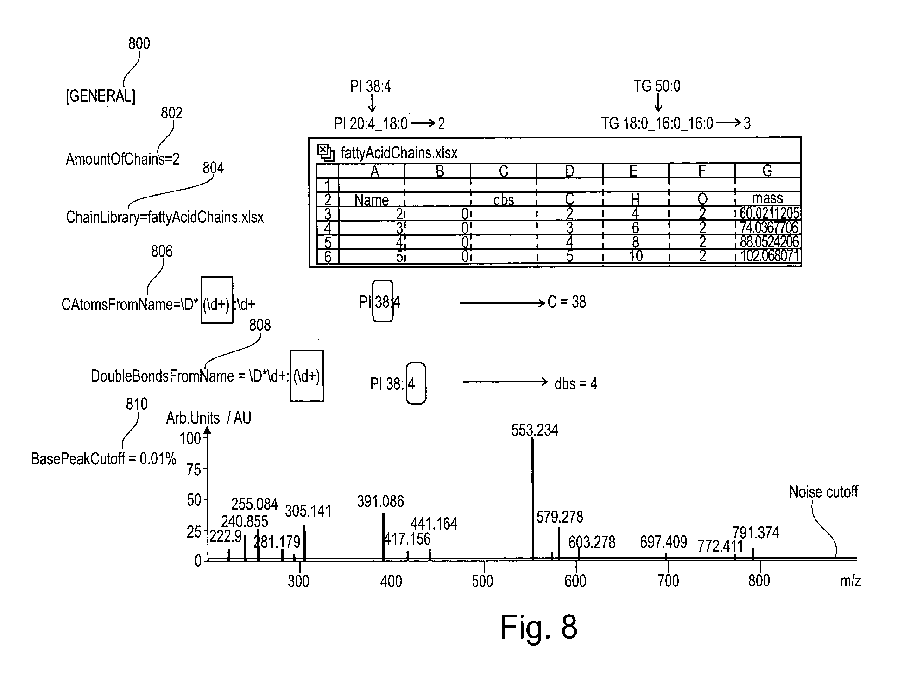

The following list exemplifies parameters, in particular required parameters, of a "GENERAL" section for a lipid class PI. An exemplary list of parameters of the "GENERAL" section 800 is presented in **Fig. 8.** The list of parameters may be a minimum required set for a class PI, and might not show a complete list of available parameters, since the parameters of the "GENERAL" section may depend on the analyte class under observation. The list of parameters may be extended to add any useful analyte class specific setup. The general parameters to setup the algorithm for PI analysis may be (see Fig. 8):

- AmountOfChains 802: This parameter may define a number of fatty acid chains attached to a backbone. For example, the number of fatty acid chains may be 2.

- ChainLibrary 804: This parameter may define a reference to a file, e.g. an Excel file, and/or a database and/or any other data source including a list or a library of potential fatty acid chains. For lipid analysis, the library may provide a number of carbon atoms, a number of double bonds, an elemental composition and/or a neutral mass value of a fatty acid chain.

- CAtomsFromName 806: This parameter may comprise a regular expression for obtaining a number of carbon atoms from a name of the analyte; e.g. for "PI 38:4", the regular expression "\D*(\d+):\d+" may return 38.

- DoubleBondsFromName 808: This parameter may comprise a regular expression for obtaining a number of double bonds from a name of the analyte; e.g. for "PI 38:4", the regular expression "\D*\d+:(\d+)" may return 4.

- BasePeakCutoff 810: This parameter may define a cutoff value relative to a base peak, i.e. the highest peak found in the spectrum. This parameter may be used to remove noisy peaks, e.g. peaks which are below a threshold of 0.01% of the base peak.

In the following, the sections "HEAD", "CHAINS", and "POSITION" are explained in detail based on the exemplary spectrum as shown in Fig. 1. In general, at least one or each section may comprise a subsection "!FRAGMENTS for a definition of fragments(rule type 1), and "!INTENSITIES" for a definition of relative signal intensities thereof (rule type 2).

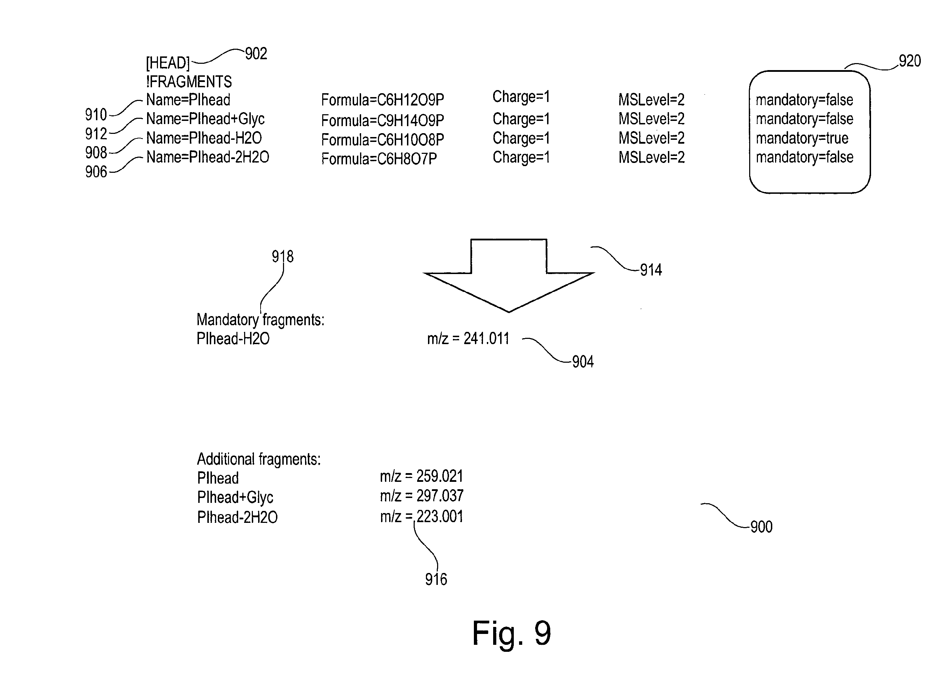

In the following, the "HEAD" section and its algorithmic processing is explained in detail. **Fig. 9** present a code example 900 of the "HEAD" section 902 and results 904 and 916 of a processing of the subsection "!FRAGMENTS" of the "HEAD" section 902. Exemplary rules to describe the fragments characteristic for a PI head group, reference numbers 102 to 108 of Fig. 1, may comprise:

*!FRAGMENTS*

*Name=PIhead Formula=C6H12O9P Charge=1 MSLevel=2 mandatory=false*

*Name=PIhead+Glyc Formula=C9H14O9P Charge=1 MSLevel=2 mandatory=false*

*Name=PIhead-H2O Formula=C6H10O8P Charge=1 MSLevel=2 mandatory=true*

*Name=PIhead-2H2O Formula=C6H8O7P Charge=1 MSLevel=2 mandatory=false.*

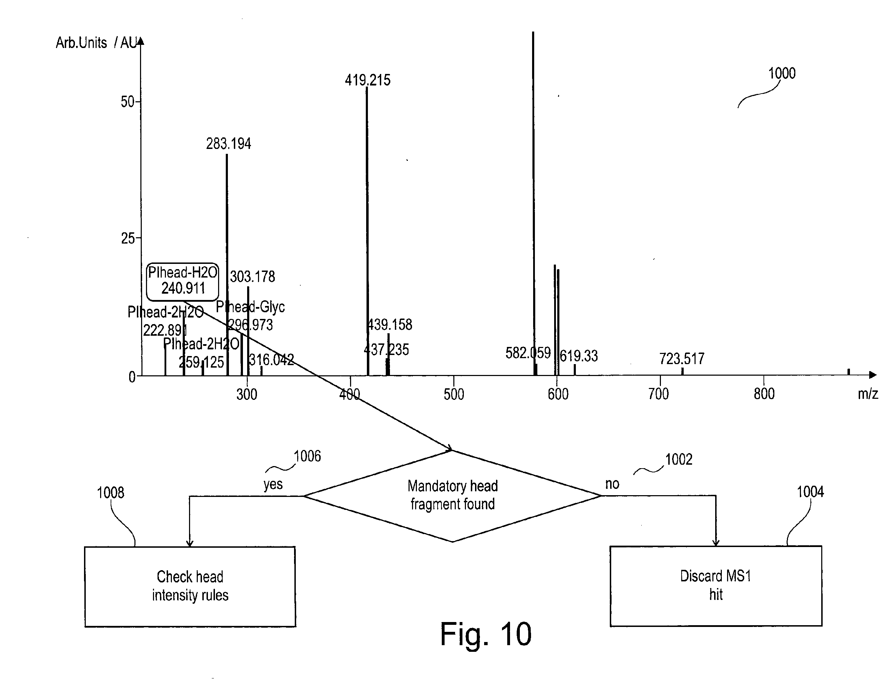

In particular, the rule "PIhead-2H2O" 906 may correspond to peak 102, the rule "PIhead-H2O" 908 may correspond to peak 104, the rule "PIhead" 910 may correspond to peak 106, and the rule "PIhead+Glyc" 912 may correspond to peak 108. The algorithm may calculate 914, for each fragment, the corresponding m/z value 904 and 916. Next, the algorithm may check for mandatory fragments 918 by processing the mandatory property 920 of the respective rule and/or by comparing the calculated m/z value 916 with one or more measured m/z values of the spectrum of the analyte, i.e. the measured spectrum of PI 38:4, to determine whether a mandatory fragment of type "HEAD" can be found 1000 as depicted in **Fig. 10.** If a mandatory fragment of type "HEAD" is missing 1002, a match may be discarded 1004 since the match might not show characteristic fragments for PI of high abundance. Next, the algorithm may check for a presence of fragments where mandatory is false. These fragments may be usually of lower abundance or may be absent. Thus, an absence of such fragment might not cause a discard of a match. These fragments are kept, e.g. stored, for further verification steps, e.g. when processing intensity rules or a spectrum intensity coverage as explained in Example 3 below. If at least one, in particular all, mandatory fragment of type "HEAD" has been found 1006, zero, one or more intensity rules of the "HEAD" section may be checked 1008.

Furthermore, some of these defined fragments may be present at higher intensities than others. Accordingly, the following optional intensity rules may be defined:

*!INTENSITIES*

*Equation=PIhead-H2O>0.1\*$BASEPEAK mandatory=true*

*Equation=PIhead-H2O>2\*PIhead mandatory=true*

*Equation=PIhead-H2O>PIhead-2H2O mandatory=true*

*Equation=PIhead+Glyc>2\*PIhead mandatory=false.*

The most prominent of the head group fragments may be "PIhead-H2O" whose intensity must be at least 10% of the highest intensity of the spectrum. The "PIhead-H2O" and the "PIhead+Glyc" may be much more intense than the "PIhead". Accordingly, at least two rules may be introduced which defines that these intensities must be twice as high as the "PIhead" intensity. Furthermore, "PIhead-H2O" may be more intense than "PIhead-2H2O" which is depicted in the third intensity rule above.

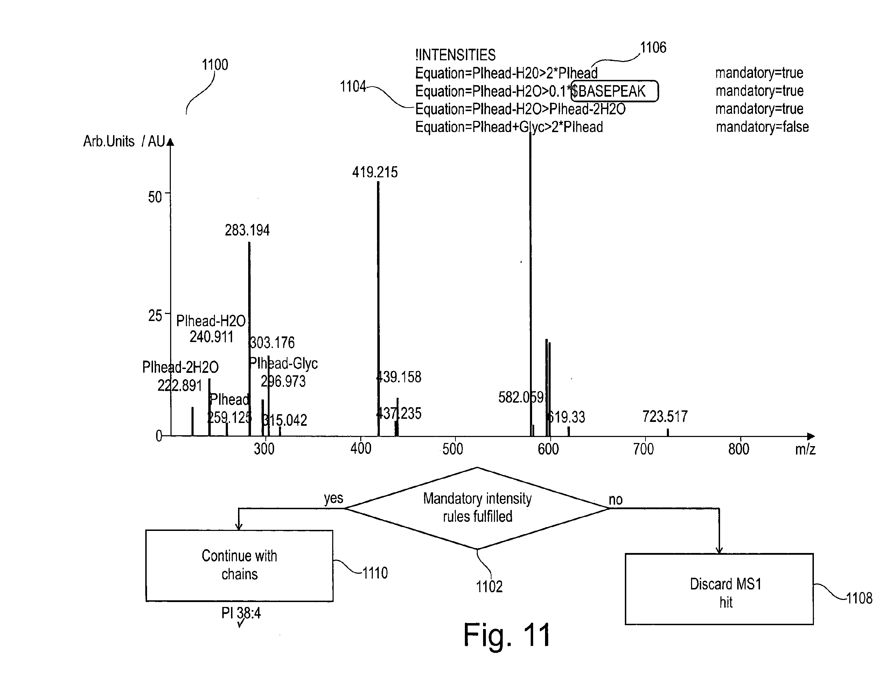

**Fig. 11** shows an overview of a processing 1100 of intensity rules (rule type 2) of the "HEAD" section of Example 2. As depicted in Fig. 11, the algorithm may check if the mandatory rules are fulfilled 1102. Only those mandatory rules may be evaluated where both of the fragments may be present. For example, if "PIhead-2H2O" has not been detected, the third rule 1104 of the set of intensity rules 1106 is not evaluated. Since analytes at small amounts do not always generate all of the fragments at detectable amounts, the evaluation of mandatory rules may be discarded for analytes which have not been detected. If a mandatory intensity rule in the "HEAD" section is violated, the match may be discarded 1108. If all of the mandatory intensity rules are met, the algorithm may check for matches where the value of the mandatory attribute of the rule is false. A violation of such rules might not entail a discard of a match. However, if the rule is met or fulfilled, it may improve the confidence of the detection. After the fragment rules and the intensity rules of the "HEAD" section are verified, the algorithm may continue 1110 with the "CHAINS" section which provides evidence for the attached fatty acid chains. From this point onwards, the rules itself may not discard the match anymore. For the example in Fig. 1, the detected MS[1] peak may be verified by MS[2] to be PI 38:4. The match may be discarded by the spectrum intensity coverage setting only, which will be explained in detail in Example 3 below. The following sections supply information about the lipid structure which might not be apparent by MS[1].

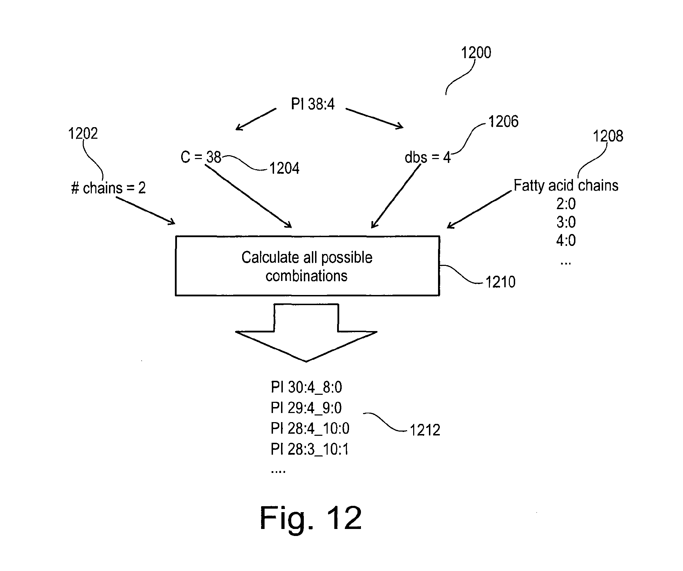

The "CHAINS" section may provide information about the fatty acid chains which may be attached to the lipid backbone. As for the "HEAD" section, the "CHAINS" section comprises a subsection for defining fragments and for relative intensity information thereof. Additionally or alternatively, the "CHAINS" section may require meta-information of the "GENERAL" section. As depicted in Fig. 12, one or more of the parameters AmountOfChains 1202, ChainLibrary 1208, CAtomsFromName 1204, and/or DoubleBondsFromName 1206 may comprise the meta-information of the "GENERAL" section. Based on the meta-information of the "GENERAL" section, the algorithm may calculate 1210 all possible permuted chain combinations fulfilling the total number of carbon atoms and the total number of double bonds of the potentially attached fatty acid chains, e.g. for PI 38:4: 30:4+8:0, 29:4+9:0, 28:4+10:0 etc., resulting in a list of potential chains 1212: 30:4, 29:4, etc. This list 1212 may represent possible variables for the $CHAIN placeholder which may be used in the fragment section of the section "CHAINS".

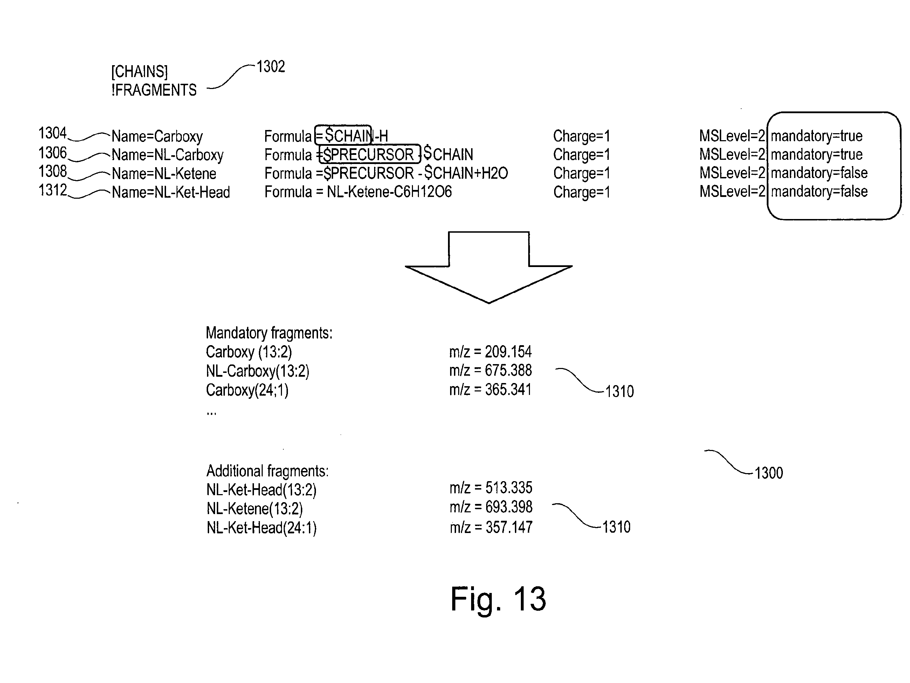

**Fig. 13** shows an overview 1300 and a processing of rules of type 1 related to chains. The rules to describe the fragments of PI originating from the chains, e.g. peaks 110 to 124 of Fig. 1 may be (see 1302 of **Fig. 13**):

*!FRAGMENTS*

*Name=Carboxy Formula=$CHAIN-H Charge=1 MSLevel=2 mandatory=true*

*Name=NL-Carboxy Formula=$PRECURSOR-$CHAIN Charge=1 MSLevel=2 mandatory=true*

*Name=NL-Ketene Formula=$PRECURSOR-$CHAIN+H2O Charge=1 MSLevel=2 mandatory=false*

*Name=NL-Ket-Head Formula=NL-Ketene-C6H12O6 Charge=1 MSLevel=2 mandatory=false.*

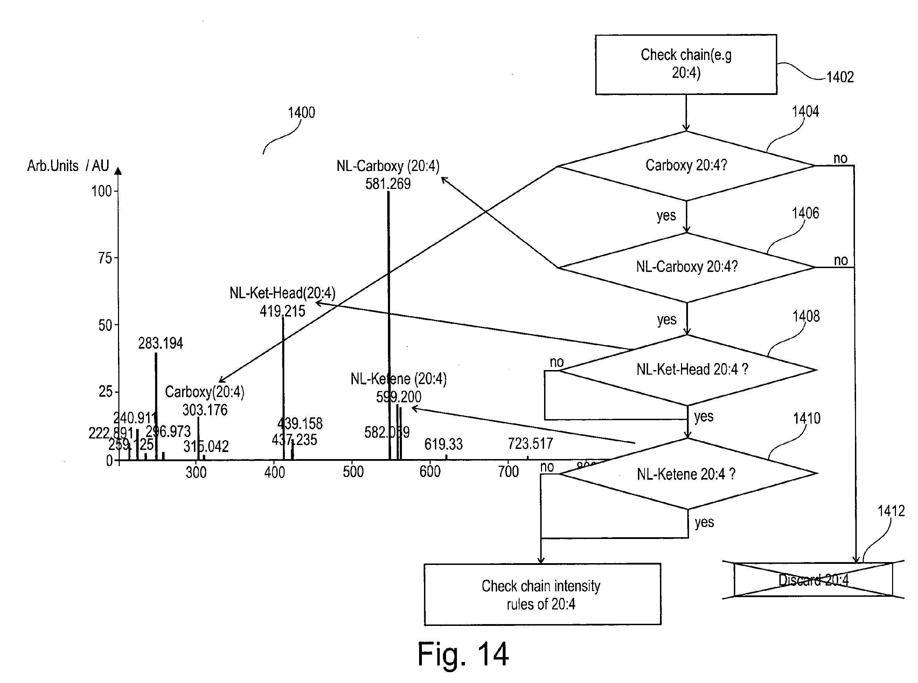

In particular, "Carboxy" 1304 may correspond to the peaks 110 and 112, "NL-Carboxy" 1306 may correspond to the peaks 114 and 118, "NL-Ketene" 1308 may correspond to the peaks 116 and 120, and "PIhead+Glyc" 1312 may correspond to the peaks 122 and 124. For each potential chain, the algorithm may calculate the corresponding m/z value 1310 for each fragment rule. As presented in **Fig. 14,** the data, in particular a generated chain of a chain combination is then checked 1402 for the existence of the mandatory fragments 1404 and 1406. Possible chains may be discarded if their mandatory fragments are not detected. For the example in Fig. 1, "Carboxy" and "NL-Carboxy" peaks may be detectable for the fatty acid chains 18:0 and 20:4 only. Consequently, all other possible chains may be discarded 1412 in this example. Next, the algorithm may check for the presence of fragments for the remaining chains 1408 and 1410 where mandatory is false. These fragments may be usually of low abundance and may be absent. Thus, an absence of such a fragment might not cause a discard of a chain. These fragments may be kept, e.g. stored, for further verification steps as defined by the intensity rules and/or the spectrum intensity coverage as explained in Example 3.

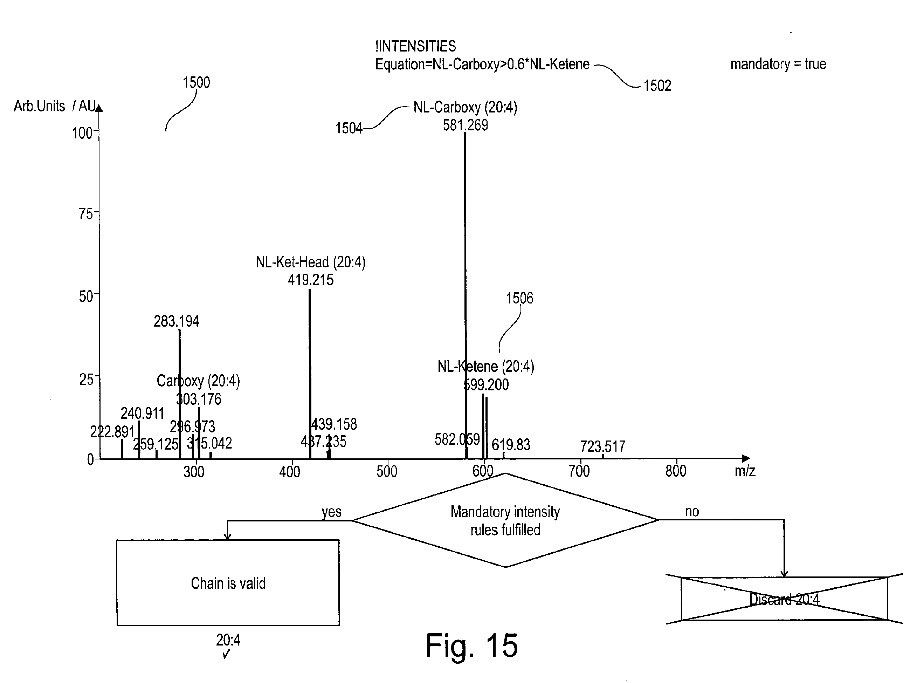

As for the "HEAD" section, a subsection about the relative intensities of the derived chain fragments may be optional for the "CHAINS" section. **Fig. 15** shows a processing 1500 of intensity rules of the "CHAINS" section. An example for the PI class may comprise the following rule (see 1502 of Fig. 15):

*!INTENSITIES*

$$Equation=NL\text{-}Carboxy>0.6*NL\text{-}Ketene\ mandatory=true.$$

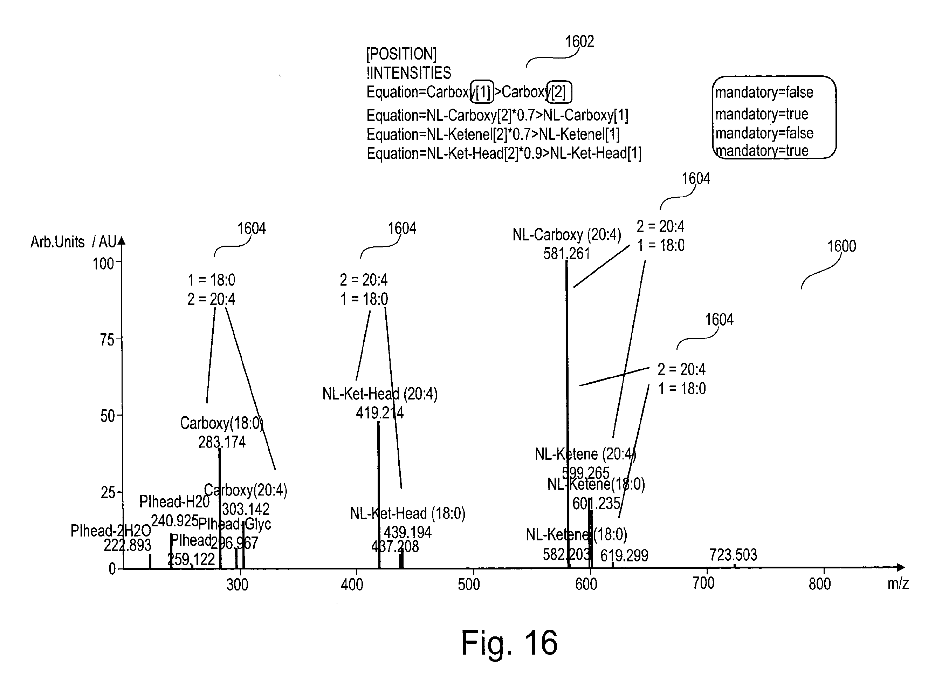

Usually, an "NL-Carboxy" fragment 1504 of PI may be observed at higher abundance than an "NL-Ketene" fragment 1506. However, in some rare cases the "NL-Ketene" fragment may show nearly the same intensity as the "NL-Carboxy" fragment. In order to support such a situation, the smaller fragment ("NL-Ketene") may be multiplied with a value smaller than one, e.g. 0.6, to provide a broader range of acceptance, and to remove accidentally matching peaks.
Fatty acid chains passing the fragmentation rules may be the input for the algorithm's decision finding process which is described in the flow chart of Fig. 2. The result of this procedure may be one or more possible chain combinations, or that there is no information about the fatty acid chains available. For the example presented in Fig. 1 and Fig. 2, the PI 38:4 species may comprise the fatty acids 18:0 and 20:4, and may be reported as PI 18:0_20:4 according to the standardized lipid nomenclature. If the "CHAINS" section reports a fatty acid combination and a "POSITION" section is present in the fragmentation rules, the algorithm may continue with an assignment of the fatty acid positions.
The "POSITION" section may provide information about a stereo-specific position of the detected fatty acids at the lipid backbone. **Fig. 16** presents a processing 1600 of the rules 1602 of the "POSITION" section. In particular, the "POSITION" section might not allow for the definition of fragments, since the stereo-specific information may be provided by a comparison of relative intensity information 1604 of chain fragments only. The following code example shows exemplary "POSITION" rules for the PI class:

*[POSITION]*

*!INTENSITIES*

$$Equation=Carboxy[1]>Carboxy[2]\ mandatory=false$$

$$Equation=NL\text{-}Carboxy[2]*0.7>NL\text{-}Carboxy[1]\ mandatory=true$$

$$Equation=NL\text{-}Ketene[2]*0.7>NL\text{-}Ketene[1]\ mandatory=false$$

$$Equation=NL\text{-}Ket\text{-}Head[2]*0.9>NL\text{-}Ket\text{-}Head[1]\ mandatory=true.$$

For these rules, the fragments defined in the "CHAINS" section may be extended by a number in square brackets. The reason is that regio-specific information may be obtained from relative intensity comparisons of fragments of the same type but originating from different fatty acid chains. Correspondingly, it may be of interest which fragment types comprise regio-specific information (not all of them do), and how the intensity relationships may change according to the position. The numbers for the positions may range from 1 to the number defined in "AmountOfChains", e.g. for PI the range 1-2 is allowed. The "POSITION" rules may be processed in two ways:

    i) if mandatory rules are present, all of the mandatory rules must be fulfilled under the assumption that referenced fragments have been found; the rules comprising "mandatory=false" may be used for additional evidence only, but might not be decisive;

    ii) if no mandatory rules are present, the algorithm may favor the position assignment where more rules match. The decision finding based on "POSITION" rules is explained in more detail in the flow chart of Fig. 3. If the "POSITION" rules return a definite decision, such as in the example in Fig. 1, the "_" in the reported lipid will be replaced by an "/" and the sequence of the fatty acids may correspond to the stereospecific position. E.g. for Figure 1, PI 18:0_20:4 is reported as PI 18:0/20:4, which means that 18:0 is at sn-1, and 20:4 is at sn-2. If no definite decision can be made, the algorithm may report PI 18:0_20:4 as final result.

In order to give an overview of the rules explained before, the complete set of rules for PI may comprise:

*[GENERAL]*

*AmountOfChains=2*

*ChainLibrary=fattyAcidChains.xlsx*

*CAtomsFromName=\D*(\d+):\d+*

*DoubleBondsFromName=\D*\d+:(\d+) BasePeakCutoff=0.01%*

*[HEAD]*

*!FRAGMENTS*

*Name=PIhead Formula=C6H12O9P Charge=1 MSLevel=2 mandatory=false*

*Name=PIhead+Glyc Formula=C9H14O9P Charge=1 MSLevel=2 mandatory=false*

*Name=PIhead-H2O Formula=C6H10O8P Charge=1 MSLevel=2 mandatory=true*

*Name=PIhead-2H2O Formula=C6H8O7P Charge=1 MSLevel=2 mandatory=false*

*!INTENSITIES*

*Equation=PIhead-H2O>2\*PIhead mandatory=true*

*Equation=PIhead-H2O>0.1\*$BASEPEAK mandatory=true*

*Equation=PIhead-H2O>PIhead-2H2O mandatory=true*

*Equation=PIhead+Glyc>2\*PIhead mandatory=false*

*[CHAINS]*

*!FRAGMENTS*

*Name=Carboxy Formula=$CHAIN-H Charge=1 MSLevel=2 mandatory=true*

*Name=NL-Carboxy Formula=$PRECURSOR-$CHAIN Charge=1 MSLevel=2 mandatory=true*

*Name=NL-Ketene Formula=$PRECURSOR-$CHAIN+H2O Charge=1 MSLevel=2 mandatory=false*

*Name=NL-Ket-Head Formula=NL-Ketene-C6H12O6 Charge=1 MSLevel=2 mandatory=false*

*!INTENSITIES*

*Equation=NL-Carboxy>0.6\*NL-Ketene mandatory=true*

*[POSITION]*

*!INTENSITIES*

*Equation=Carboxy[1]>Carboxy[2] mandatory=false*

$$Equation=NL\text{-}Carboxy[2]*0.7>NL\text{-}Carboxy[1]\ mandatory=true$$

$$Equation=NL\text{-}Ketene[2]*0.7>NL\text{-}Ketene[1]\ mandatory=false$$

$$Equation=NL\text{-}Ket\text{-}Head[2]*0.9>NL\text{-}Ket\text{-}Head[1]\ mandatory=true.$$

In Example 2 as described above, the usage of the two types of rules is described constituting the fragmentation language, i.e. rules for the description of fragments and rules for relative intensities thereof. Further, it has been shown how these rules may be applied to gain structural information based on the example of the lipid PI 38:4. With these rules, fragmentation processes for metabolites may be depicted without writing specific algorithms for each class of analytes. Furthermore, users can easily adapt the rules to their experimental setup and analyte class under observation without specific knowledge about the algorithm which processes these rules and finds decisions based on them. In fact, the rules may be a textual description of the visual spectrum, which therefore can be easily compared and adapted, since the rules follow a natural understanding of fragments in mass spectrometry.

**EXAMPLE 3: specificity without head group fragments and partial position assignment at the example of TG (triacylglycerols) in positive ion mode**

The rules may allow for a verification of MS[1] identifications based on one or more head group fragments, whereas one or more chain fragments may reveal information about the structure. However, there are analyte classes that might not have one or more fragments characteristic for the analyte class, or they might not occur frequently throughout the spectra. Such an analyte class may be the group of triacylglycerols. This lipid class may comprises three fatty acids at a glycerol, and may be in positive ion mode observed usually with an $NH_4$ adduct. There may be only one fragment characteristic for this class which occurs infrequently, i.e. the neutral loss of $NH_3$. Consequently, this fragment might not be well-suited for specificity improvement, since it would dramatically increase the false negative rate because of its infrequent occurrence. However, the fragments originating from the losses of fatty acid chains may be of high abundance and may cover the highest intensities of the spectrum (see e.g. Fig. 4). Thus, an additional parameter might be added to the "GENERAL" section, i.e. the parameter "SpectrumCoverage", which specifies a relative amount of total intensities that must be covered by found fragments defined in the rules, e.g. 35%. The suggested fragmentation rules for TG class may be:

[GENERAL]

$$AmountOfChains=3$$

$$ChainLibrary=fattyAcidChains.xlsx$$

$$CAtomsFromName=\backslash D*(\backslash d+):\backslash d+$$

$$DoubleBondsFromName=\backslash D*\backslash d+:(\backslash d+)$$

$$BasePeakCutoff=0.01\%$$

*SpectrumCoverage=50%*

*[HEAD]*

*!FRAGMENTS*

*Name=NH3-Loss Formula=$PRECURSOR-NH3 Charge=1 MSLevel=2 mandatory=false*

*[CHAINS]*

*!FRAGMENTS*

*Name=NL-Carboxy Formula=$PRECURSOR-$CHAIN-NH3 Charge=1*

*MSLevel=2 mandatory=true*

*!INTENSITIES*

*Equation=NL-Carboxy>0.1\*$BASEPEAK mandatory=true*

*[POSITION]*

*!INTENSITIES*

*Equation=0.8\*NL-Carboxy[1]>NL-Carboxy[2] mandatory=false*

*Equation=0.8\*NL-Carboxy[3]>NL-Carboxy[2] mandatory=false.*

In contrast to the PI rules, the "HEAD" fragment might not be mandatory, and even the total "HEAD" section may be omitted, since the quality criterion for a good identification is satisfied by the spectrum intensity coverage, where all of the found fragments contribute to. The "NH3-Loss" may be defined only to have an additional peak that may contribute to the spectrum intensity coverage.

A peculiarity of the lipid class TG may be a lack of a possibility for a complete stereospecific assignment of the fatty acid chains at the glycerol backbone, since the observed fragments of the sn-1 and sn-3 position yield comparable intensities. Only the neutral loss of the fatty acid at sn-2 may be less probable, and as such detectable. For such a case, two rules may be defined which compare the sn-1 and sn-3 position to sn-2. Since there might not be a complete assignment of the position, the algorithm may report the TG in Fig. 4 as "16:0/18:1/18:0; 18:0/18:1/16:0" indicating more than one possible interpretation of the spectrum.

Advantageously, the output of the rules may be easily extended to further quality criteria such as a spectrum intensity coverage. Further the sections of the rules might not be necessarily mandatory (e.g. it would be possible to omit the "HEAD") section leading to a flexible adaptation of the rules to new analytes. In addition, it is possible to make partial assignments of the fatty acid positions.

**EXAMPLE 4: specificity with different head group fragments and exclusive fragment at the example of PC (phosphatidylcholin) in positive ion mode**

In positive ion mode, the strongest measurable adduct form may be usually a protonated PC. This analyte class may usually exhibit a very strong characteristic head group fragment at 184 Th (see e.g. peak 502 of spectrum 504 of Fig. 5). For some mass spectrometry setup, however, this fragment may be out of a detection range (see e.g. 506 of Fig. 5). Consequently, this fragment might not be used as a mandatory fragment, despite its prominence in many of the spectra. Nevertheless, for spectra where 184 Th may be out of range, another, but not so prominent loss may be observed, i.e. the neutral loss of nitrogen and three methyl groups (-NC$_3$H$_9$ = "NL-triMeth"; see e.g. peak 510 of spectrum 506 of Fig. 5). These two fragments in combination with the spectrum intensity coverage may allow a reliable detection of PC species. However, PC in positive ion mode may form in addition to a protonated adduct lower amounts of sodiated adducts. The difficulty may be that the sodiated form is nearly isobar to the protonated form with two more carbon atoms in the fatty acid chains and three more double bonds (e.g. protonated PC 34:4) may have two more carbon atoms in the chain, and three more double bonds:

$$2 \text{ carbon atoms in chain} = +2C+4H; +3 \text{ double bonds} = -6H \rightarrow \text{mass}$$
$$\text{difference between PC 32:1 and PC 34:4} = +2C-2H \approx 24Da - 2Da = 22Da;$$

$$\text{sodiated} = +Na; \text{ protonated} = +H \rightarrow \text{mass difference in adducts} = +Na-H \approx$$
$$23Da - 1Da = 22Da.$$

Furthermore, the sodiated form may show as well a "NL-triMeth" fragment (see e.g. peak 510 of spectrum 508 of Fig. 5) which may make an exclusion of this false positive based on spectrum intensity coverage unfeasible. However, the sodiated form might not exhibit a head group fragment, but its neutral loss (-183 Da, see "NL-Na_wrong" peak 514 of spectrum 508 of Fig. 5) which may be scarcely observed in protonated fragmentation spectra. Hence, such fragments can be excluded by defining this fragment and setting the criterion for a good match. For example, the criterion may be defined as the intensity of this fragment being below a certain value. As an example of how to depict such complex relationship, the following rules may be used:

*[GENERAL]*

$$AmountOfChains=2$$

$$ChainLibrary=fattyAcidChains.xlsx$$

$$CAtomsFromName=\backslash D*(\backslash d+):\backslash d+$$

*DoubleBondsFromName=\D*\d+:(\d+)*

*BasePeakCutoff=0.02%*

*SpectrumCoverage=10%*

*[HEAD]*

*!FRAGMENTS*

*Name=PChead Formula=P1O4C5N1H15 Charge=1 MSLevel=2 mandatory=false*

*Name=Precursor Formula=$PRECURSOR Charge=1 MSLevel=2 mandatory=false*

*Name=NL-triMeth Formula=$PRECURSOR-NC3H9 Charge=1 MSLevel=2 mandatory=false*

*Name=NL-Na_wrong Formula=$PRECURSOR-P1O4C5N1H14 Charge=1 MSLevel=2 mandatory=false*

*!INTENSITIES*

*Equation=PChead>0.6*$BASEPEAK mandatory=true*

*Equation=NL-Na_wrong<0.4*$BASEPEAK mandatory=true*

*[CHAINS]*

*!FRAGMENTS*

*Name=NL-Ketene Formula=$PRECURSOR-$CHAIN+H2O Charge=1 MSLevel=2 mandatory=false*

*Name=NL-Carboxy Formula=$PRECURSOR-$CHAIN Charge=1 MSLevel=2 mandatory=true*

*[POSITION]*

*!INTENSITIES*

*Equation=NL-Ketene[2]\*0.6>NL-Ketene[1] mandatory=true.*

The fragmentation rule "PChead" may account for a main head group fragment at 184 Th, but it might not be flagged as mandatory, thereby preventing a removal of PC species such as depicted in 506 of Fig. 5. However, the intensity rule of "PChead" may guarantee for high specificity if the fragment is observed. The "NL-triMeth" and the "Precursor" fragment in combination with the "SpectrumCoverage" may account for PC species where the characteristic head fragment may be out of the measurement range, such as in 506 of Fig. 5. The fragment "NL-Na_wrong" may detect head group losses of the sodiated form, and its intensity rule may guarantee the removal of sodiated spectra, since the rule may accept these peaks only if these peaks are below a certain threshold. In this manner, spectra such as the one in 508 of Fig. 5 may be removed.

The example 4 shows that even complex fragmentation relations may be depicted with the presented fragmentation rules. The advantage may be that all of these specific adaptions to different molecule classes may be implemented without changing any single line of code.

**EXAMPLE 5: detection of different fatty acid types (mixture of acyl-, alkyl- , or alkenyl-linked fatty acids) at the example of O-PE in negative ion mode**

Some lipid subclasses may comprise fatty acid chains which may be linked to a glycerol backbone in different manner, e.g. 1-alkyl,2-acylglycerophosphoethanolamines (O-PE) which may comprise one acyl linked fatty acid and one alkyl linked fatty acid. Even though such fragments may be reflected with the previously presented rules, the algorithm might lose specificity, since only a combination of fatty acid fragments comprising one alkyl- and one acyl-linked fatty acid may be valid, and such case might not be covered by the check "Is any combination possible" in the flow diagram of Fig. 2. In order to cover such cases, the "GENERAL" section may allow for the non mandatory parameters "'AlkylChains" and "AlkenylChains". If no such parameter is entered, the algorithm may assume that every fatty acid chain may be acyl linked. If the parameters "AlkylChains" and "AlkenylChains" are added to the "GENERAL" section, the parameter may automatically reduce the amount of remaining acyl chains. In order to reflect the alkyl-linked and alkenyl-linked chains in the fragmentation rules, two additional placeholders may be used, namely "$ALKYLCHAIN" and "$ALKENYLCHAIN".

A complete set of rules for O-PE may comprise:

*[GENERAL]*

*AmountOfChains=2*

ChainLibrary=fattyAcidChains.xlsx

CAtomsFromName=\D*(\d+):\d+

DoubleBondsFromName=\D*\d+:(\d+)

BasePeakCutoff=0.01%

SpectrumCoverage=10%

AlkylChains=1

[CHAINS]

!FRAGMENTS

Name=Carboxy Formula=$CHAIN-H Charge=1 MSLevel=2mandatory=true

Name=Carb-Alk Formula=$ALKYLCHAIN-H Charge=1 MSLevel=2
  mandatory=false

Name=NL-Carboxy Formula=$PRECURSOR-$CHAIN Charge=1 MSLevel=2
mandatory=false

Name=NL-Ketene Formula=$PRECURSOR-$CHAIN+H2O Charge=1
MSLevel=2 mandatory=false

!INTENSITIES

*Equation=1.2\*Carboxy>NL-Ketene mandatory=true*

*Equation=0.2\*Carboxy>Carb-Alk mandatory=true.*

PE may have two attached fatty acid chains which may be reflected by the parameter "AmountOfChains=2". The parameter "AlkylChains=1" may specify that one fatty acid chain is an alkyl linked fatty acid chain. Thus, the remaining fatty acid chain may be acyl linked. The "Carb-Alk" fragment may account for a fragment originating from the alkyl linked fatty acid chain because of the "$ALKYLCHAIN" placeholder. As defined in the second line of the intensity rules, the equation comprises an intensity comparison between an acyl linked fragment and an alkyl linked fragment. Usually, the intensity comparisons of the "CHAINS" section may be performed only on fragments from the same fatty acid chain (e.g. 18:0). However, in this case, the comparison may be conducted on a fatty acid combination (e.g. O-18:0 and 18:1).

Example 5 shows that the principle of defining fragments and rules for relative intensities thereof may be extended to other use cases. For other use cases, the "GENERAL" section may be extended accordingly; new placeholders and possibly new sections may be introduced. However, the basic principle may be applied to any analyte classes for mass spectra comprising fragments.

### EXAMPLE 6: isobaric species - mixed spectra

For many metabolomic analyte classes, there may be isobaric species. Such species might not be separated by pre-separation methods such as liquid chromatography. Thus, classes comprising isobaric species with similar physicochemical properties such as lipids may be concealed in a single peak in MS[1] identifications. Consequently, the occurrence of spectra originating from more than one species may be the rule rather than the exception for lipids. Such cases may be covered by the fragmentation language of the present application (see e.g. Fig. 6). The chain rules presented in Example 2 may allow for more than one fatty acid combination to be reported. For the reported species, all of the intensities of fragments of type "CHAIN" belonging to one species may be summed. The intensity determined by MS[1] may be split among determined species corresponding to these sums. The assumption behind this split is that the same fragment types should have approximately the same fragmentation efficiencies, and thus should be comparable. Nevertheless, such splits may deliver only rough estimates of the real proportions among isobaric species, since fragmentation spectra for the same precursor might not be acquired as often as MS[1] spectra, since several precursor masses may be selected for fragmentation. Hence, quantitative evidence based on fragmentation spectra may suffer generally from undersampling. In order to get rid of very minor chain contributions, the property "ChainCutoff" of the "GENERAL" section may be used. In other words, the property "ChainCutoff" may provide a filter for chains. For this filter, the "CHAIN" sums are used. The property "ChainCutoff" may specify a threshold relative to the highest detected sum (fragments of fatty acid chains belonging to one combination). If one sum is below this cutoff value, this species may be discarded. For example, the property "ChainCutoff" may be set to a value of 1%, in order to be quite sensitive to minor contributions. However, for spectra where there are scarcely any "CHAIN" fragments observed and where these are close to the noise level, such as for PC and PE (phosphatidylethanolamine) in positive ion mode, it is better to increase this value to higher values such as 50%, in order to get rid of accidentally matching fragment combinations based on noise peaks.

### EXAMPLE 7: intensity extraction

Generally, there might be more than one fragmentation spectrum present for each peak identified in MS[1]. Principally, any kind of algorithm may be possible to extract intensity values out of the spectra for further processing based by the presented fragmentation rules. Even a structural assessment based on each individual spectrum may be performed. To obtain a single intensity value for each fragment out of several spectra, the algorithm may accept a settable m/z range around a calculated m/z value and sums all of the intensities within this m/z range to a total intensity, whereupon only fragmentation spectra may be selected which originate from the m/z value of the intact species and are within the time borders of the MS[1] identification. Thus, fragmentation spectra closer to the MS[1] peak center in the time dimension may be selected which are usually more intense and less tampered with other species. Advantageously, more reliable spectra may automatically receive higher emphasis. Furthermore, the proposed method is extremely fast.

Additionally, a chromatographic extraction for verified fatty acid chain fragments may be performed for a more detailed analysis of sn positions, since the total intensity of a fragments may favor sn assignments of the more intense species. Typically, pre-separation methods such as liquid chromatography may separate even isomeric species slightly. Even though such separations may not be detectable on the MS[1] level, the difference in the fragmentation efficiencies

on the MS$^n$ level may be exploited to detect the presence of e.g. both species comprising the same fatty acids, but at different sn positions. In Fig. 7, the detection of both species, PC 16:0/18:1 and PC 18:1/16:0, is shown which originate from one MS1 peak of PC 34:1 based on the chromatographic approach. Nevertheless, even with this approach the detection of two isomeric substances may be limited. A detection of both species might not be possible if the chromatographic separation might not be sufficient, or one species may be much smaller than the other one, since the intensities of the smaller species may be lost during smoothing.

It should be noted that the term "comprising" does not exclude other elements or steps and the use of articles "a" or "an" does not exclude a plurality. Also elements described in association with different embodiments may be combined. It should also be noted that reference signs in the claims should not be construed as limiting the scope of the claims.

**Claims**

1. Computer implemented method for automatically determining fragments in a sample, the method comprising:

   receiving a set of values of a measurement of an analyte composition in the sample;
   determining at least one fragment that corresponds to at least one value of the set of values by processing at least one rule of a plurality of rule definitions,
   wherein the at least one rule defines a fragment information, and
   wherein the fragment information of the at least one rule defines a value that corresponds to the at least one value of the set of values; and
   identifying the at least one fragment by the fragment information of the at least one rule.

2. Method as set forth in the preceding claim, wherein, for each fragment, at least one rule is defined in the plurality of rule definitions.

3. Method as set forth in any one of the preceding claims, wherein the set of values of the measurement corresponds to MS$^n$ spectra of the analyte composition, and/or
   wherein the at least one fragment is identified in a first spectrum of the MS$^n$ spectra and a different fragment is identified in a different spectrum of the MS$^n$ spectra.

4. Method as set forth in any one of the preceding claims, wherein the analyte composition comprises at least one metabolite species.

5. Method as set forth in any one of the preceding claims, wherein the set of rules comprises at least one rule of a first type and/or at least one rule of a second type, wherein the at least one rule of the first type defines fragment information and the at least one of rule of the second type defines intensity information.

6. Method as set forth in claim 5, wherein the processing of at least one rule of the first type comprises:

   determining at least one of a head fragment and/or a chain fragment based on the fragment information.

7. Method as set forth in any one of claims 5 to 6, wherein the processing of at least one rule of the second type comprises:

   checking whether at least one rule of the second type is defined,
   if at least one rule of the second type is defined,
   determining whether the intensity information of the at least one rule of the second type is fulfilled by the set of values of the measurement to verify at least one of a head fragment, a chain fragment, and/or a position of the chain fragment; and
   identifying the at least one fragment by the fragment information of the at least one rule of the first type and the intensity information of the at least of rule of the second type.

8. Method as set forth in any one of claims 5 to 7, wherein the intensity information is defined relative to one or more fragments as defined by the fragment information of the at least one rule of the first type.

9. Method as set forth in any one of the preceding claims, wherein the plurality of rule definitions defines at least one rule for at least one of a head fragment, a fatty acid chain, and/or a position of a fatty acid chain.

**10.** Method as set forth in any one of the preceding claims, wherein the at least one rule defines at least one fragment of the analyte composition relative to a placeholder, wherein the placeholder comprises at least one of a precursor, an acyl chain, an alkyl chain, and/or an alkenyl chain.

**11.** Method as set forth in any one of the preceding claims, wherein the at least one rule comprises a property to define whether the at least one rule is mandatory.

**12.** Method as set forth in any one of the preceding claims, the method further comprising:

filtering the set of values based on a predefined threshold value prior to determining the at least one fragment.

**13.** Method as set forth in any one of the preceding claims, the method further comprising:

providing structural information of the analyte composition regarding a head fragment, one or more fatty acid chains, and/or a positional information of the one or more fatty acid chains as a result.

**14.** Computer system for automatically determining fragments in a sample, the system comprising:

a memory configured to store computer-readable instructions,
a processor configured to process the computer-readable instructions to perform a method of any one of claims 1 to 13.

**15.** Computer-readable medium, the computer-readable medium comprising instructions which, when executed on a computer system, perform a method according to any one of claims 1 to 14.

EP 2 998 891 A1

Fig. 1

200

Found chains: 18:0, 18:1,
20:3, 20:4, 22:4 — 202

204

Any chains found ? — no

yes

208

Is any combination possible ? — no

yes

20:4_18:0, 20:3_18:1 — 210

Filter out minor contributions
(e.g. < 1%) — 212

214

Continue with
position

206

No chain information available

PI 20:4_18:0
✓

PI 38:4
✓

# Fig. 2

EP 2 998 891 A1

300

PI 20:4_18:0 — 302

318

More additional rule evidence? — no — Are there mandatory rules? 304 — yes — Mandatory rules fulfilled? 306 — no

no

yes

yes

310

Assign distinct identifications — Check for additional rules — 316

312

Assign multiple recommendations (higher evidence)

314

PI 18:0 / 20:4
✓

308

No position assignment possible

PI 20:4_18:0
✓

Fig. 3

Fig. 4

EP 2 998 891 A1

Fig. 5

Fig. 6

Fig. 7

31

800

[GENERAL]

802

AmountOfChains=2

804

ChainLibrary=fattyAcidChains.xlsx

806

CAtomsFromName=\D* (\d+) :\d+

808

DoubleBondsFromName = \D*\d+: (\d+)

810

BasePeakCutoff = 0.01%

PI 38:4
↓
PI 20:4_18:0 ⟶ 2

TG 50:0
↓
TG 18:0_16:0_16:0 ⟶ 3

fattyAcidChains.xlsx

| | A | B | C | D | E | F | G |
|---|---|---|---|---|---|---|---|
| 1 | | | | | | | |
| 2 | Name | | dbs | C | H | O | mass |
| 3 | 2 | 0 | | 2 | 4 | 2 | 60.0211205 |
| 4 | 3 | 0 | | 3 | 6 | 2 | 74.0367706 |
| 5 | 4 | 0 | | 4 | 8 | 2 | 88.0524206 |
| 6 | 5 | 0 | | 5 | 10 | 2 | 102.068071 |

PI 38:4 ⟶ C = 38

PI 38:4 ⟶ dbs = 4

Arb.Units / AU

Noise cutoff

553.234

391.086

255.084
240.855   305.141
222.9   281.179   417.156   441.164   579.278   603.278   697.409   772.411   791.374

300   400   500   600   700   800   m/z

Fig. 8

[HEAD] ⁓902
!FRAGMENTS

| | | | | |
|---|---|---|---|---|
| Name=Plhead | Formula=C6H12O9P | Charge=1 | MSLevel=2 | mandatory=false |
| Name=Plhead+Glyc | Formula=C9H14O9P | Charge=1 | MSLevel=2 | mandatory=false |
| Name=Plhead-H2O | Formula=C6H10O8P | Charge=1 | MSLevel=2 | mandatory=true |
| Name=Plhead-2H2O | Formula=C6H8O7P | Charge=1 | MSLevel=2 | mandatory=false |

910
912
908
906

920

918

Mandatory fragments:
Plhead-H2O                     m/z = 241.011 ⁓904

914

Additional fragments:
Plhead            m/z = 259.021
Plhead+Glyc       m/z = 297.037
Plhead-2H2O       m/z = 223.001

916

900

Fig. 9

EP 2 998 891 A1

Fig. 10

Fig. 11

1200

PI 38:4

1202

C = 38 —1204

dbs = 4 —1206

1208

# chains = 2

Fatty acid chains
2:0
3:0
4:0
...

Calculate all possible
combinations

—1210

PI 30:4_8:0
PI 29:4_9:0
PI 28:4_10:0
PI 28:3_10:1
....

—1212

# Fig. 12

[CHAINS] — 1302
!FRAGMENTS

| | | | | |
|---|---|---|---|---|
| 1304 Name=Carboxy | Formula =$CHAIN-H | Charge=1 | MSLevel=2 | mandatory=true |
| 1306 Name=NL-Carboxy | Formula =$PRECURSOR -$CHAIN | Charge=1 | MSLevel=2 | mandatory=true |
| 1308 Name=NL-Ketene | Formula =$PRECURSOR -$CHAIN+H2O | Charge=1 | MSLevel=2 | mandatory=false |
| 1312 Name=NL-Ket-Head | Formula = NL-Ketene-C6H12O6 | Charge=1 | MSLevel=2 | mandatory=false |

Mandatory fragments:
Carboxy (13:2)         m/z = 209.154
NL-Carboxy(13:2)       m/z = 675.388
Carboxy(24;1)          m/z = 365.341     —1310
...

Additional fragments:
NL-Ket-Head(13:2)      m/z = 513.335
NL-Ketene(13:2)        m/z = 693.398
NL-Ket-Head(24:1)      m/z = 357.147     —1310

—1300

Fig. 13

Fig. 14

Fig. 15

Fig. 16

Europäisches
Patentamt
European
Patent Office
Office européen
des brevets

# EUROPEAN SEARCH REPORT

Application Number

EP 14 18 5566

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SONG H. ET AL: "Algorithm for Processing Raw Mass Spectrometric Data to Identify and Quantitate Complex Lipid Molecular Species in Mixtures by Data-Dependent Scanning and Fragment Ion Database Searching", JOURNAL OF THE AMERICAN SOCIETY FOR MASS SPECTROMETRY, ELSEVIER SCIENCE INC, US, vol. 18, no. 10, 20 September 2007 (2007-09-20), pages 1848-1858, XP022262078, ISSN: 1044-0305, DOI: 10.1016/J.JASMS.2007.07.023 * abstract * * page 1848, right-hand column, line 28 - page 1849, left-hand column, line 14 * * page 1849, right-hand column, lines 15-41 * * page 1851, left-hand column, lines 1-5 * * table 1 * | 1-15 | INV. G06F19/00 |
| X | US 2008/167824 A1 (REINHOLD VERNON N [US] ET AL) 10 July 2008 (2008-07-10) * abstract * * figures 2,4 * * paragraphs [0010], [0011], [0099], [0100] * * paragraph [0137] - paragraph [0144] * | 1-15 | TECHNICAL FIELDS SEARCHED (IPC) G06F |
| X | US 2005/065738 A1 (RAGURAM SASISEKHARAN [US]) 24 March 2005 (2005-03-24) * abstract * * figures 2,3,6,7 * * paragraphs [0012], [0014], [0046], [0057], [0136] * | 1-15 | |

-/--

The present search report has been drawn up for all claims

| Place of search | Date of completion of the search | Examiner |
|---|---|---|
| The Hague | 10 February 2015 | Godzina, Przemyslaw |

EPO FORM 1503 03.82 (P04C01)

Europäisches
Patentamt

European
Patent Office

Office européen
des brevets

## EUROPEAN SEARCH REPORT

Application Number

EP 14 18 5566

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 741 223 A1 (THERMO FINNIGAN LLC [US]) 11 June 2014 (2014-06-11)<br>* abstract *<br>* paragraphs [0003], [0020] *<br>* paragraph [0041] - paragraph [0044] *<br>----- | 1-15 | |
| X | DRAPER J. ET AL: "Metabolite signal identification in accurate mass metabolomics data with MZedDB, an interactive m/z annotation tool utilising predicted ionisation behaviour 'rules'",<br>BMC BIOINFORMATICS, BIOMED CENTRAL, LONDON, GB,<br>vol. 10, no. 1, 21 July 2009 (2009-07-21), page 227, XP021055673,<br>ISSN: 1471-2105, DOI: 10.1186/1471-2105-10-227<br>* abstract *<br>* page 4, left-hand column, lines 24-32 *<br>* page 5, left-hand column, line 5 - page 6, left-hand column, line 17 *<br>* page 8, left-hand column, lines 11-14 *<br>* page 10, right-hand column, lines 26-35 *<br>* table 2 *<br>* figure 2 *<br>-----<br>-/-- | 1-15 | |

**TECHNICAL FIELDS SEARCHED (IPC)**

The present search report has been drawn up for all claims

| Place of search | Date of completion of the search | Examiner |
|---|---|---|
| The Hague | 10 February 2015 | Godzina, Przemyslaw |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

# EUROPEAN SEARCH REPORT

Application Number

EP 14 18 5566

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WOLF S. ET AL: "In silico fragmentation for computer assisted identification of metabolite mass spectra", BMC BIOINFORMATICS, BIOMED CENTRAL, LONDON, GB, vol. 11, no. 1, 22 March 2010 (2010-03-22), page 148, XP021071491, ISSN: 1471-2105, DOI: 10.1186/1471-2105-11-148 * abstract * * page 3, right-hand column, line 19 - page 4, left-hand column, line 2 * * page 4, right-hand column, line 19 - page 5, left-hand column, line 3 * * table 1 * ----- | 1-15 | |

TECHNICAL FIELDS
SEARCHED      (IPC)

The present search report has been drawn up for all claims

| Place of search | Date of completion of the search | Examiner |
|---|---|---|
| The Hague | 10 February 2015 | Godzina, Przemyslaw |

EPO FORM 1503 03.82 (P04C01)

**EP 2 998 891 A1**

## ANNEX TO THE EUROPEAN SEARCH REPORT
## ON EUROPEAN PATENT APPLICATION NO.

EP 14 18 5566

This annex lists the patent family members relating to the patent documents cited in the above-mentioned European search report.
The members are as contained in the European Patent Office EDP file on
The European Patent Office is in no way liable for these particulars which are merely given for the purpose of information.

10-02-2015

| Patent document cited in search report | | | Publication date | Patent family member(s) | | | Publication date |
|---|---|---|---|---|---|---|---|
| US | 2008167824 | A1 | 10-07-2008 | US<br>WO | 2008167824<br>2008027599 | A1<br>A2 | 10-07-2008<br>06-03-2008 |
| US | 2005065738 | A1 | 24-03-2005 | US<br>US<br>US<br>US<br>WO | 2005065738<br>2009043513<br>2011159476<br>2012258875<br>2005026720 | A1<br>A1<br>A1<br>A1<br>A1 | 24-03-2005<br>12-02-2009<br>30-06-2011<br>11-10-2012<br>24-03-2005 |
| EP | 2741223 | A1 | 11-06-2014 | EP<br>US | 2741223<br>2014138531 | A1<br>A1 | 11-06-2014<br>22-05-2014 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013126725 A

**Non-patent literature cited in the description**

- **LIEBISCH et al.** *LipidMaps, Journal of Lipid Research,* vol. 54 (6), 1523-30